(19) **Europäisches Patentamt / European Patent Office / Office européen des brevets**

(11) **EP 3 362 041 B1**

(12) # EUROPEAN PATENT SPECIFICATION

(45) Date of publication and mention
of the grant of the patent:
**08.04.2026 Bulletin 2026/15**

(21) Application number: **16788338.8**

(22) Date of filing: **14.10.2016**

(51) International Patent Classification (IPC):
**A61K 9/00** (2006.01)    **A61K 9/14** (2006.01)
**A61K 9/19** (2006.01)    **A61P 43/00** (2006.01)

(52) Cooperative Patent Classification (CPC):
**A61K 9/146; A61K 9/0051; A61K 9/19; A61P 43/00**

(86) International application number:
**PCT/US2016/057019**

(87) International publication number:
**WO 2017/066554 (20.04.2017 Gazette 2017/16)**

(54) **STABLE PROTEIN COMPOSITIONS**

**STABILE PROTEINZUSAMMENSETZUNGEN**

**COMPOSITIONS PROTÉINIQUES STABLES**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.10.2015 US 201562242412 P**

(43) Date of publication of application:
**22.08.2018 Bulletin 2018/34**

(73) Proprietor: **Regeneron Pharmaceuticals, Inc.
Tarrytown, NY 10591 (US)**

(72) Inventors:
• **CHEN, Hunter**
**Tarrytown, New York 10591 (US)**
• **SCHLESINGER, Erica**
**Tarrytown, New York 10591 (US)**

(74) Representative: **J A Kemp LLP
80 Turnmill Street
London EC1M 5QU (GB)**

(56) References cited:
**US-A1- 2012 095 439**

• SANDRA HERRMANN ET AL: "New Insight into the Role of Polyethylene Glycol Acting as Protein Release Modifier in Lipidic Implants", PHARMACEUTICAL RESEARCH, KLUWER ACADEMIC PUBLISHERS-PLENUM PUBLISHERS, NL, vol. 24, no. 8, 23 March 2007 (2007-03-23), pages 1527 - 1537, XP019507255, ISSN: 1573-904X, DOI: 10.1007/S11095-007-9271-Y

• ATHA D H ET AL: "MECHANISM OF PRECIPITATION OF PROTEINS BY POLY ETHYLENE GLYCOLS ANALYSIS IN TERMS OF EXCLUDED VOLUME", JOURNAL OF BIOLOGICAL CHEMISTRY.(MICROFILMS), AMERICAN SOCIETY OF BIOLOGICAL CHEMISTS, BALTIMORE, MD, US, vol. 256, no. 23, 1 January 1981 (1981-01-01), pages 12108 - 12117, XP002454548

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD**

The invention generally pertains to compositions and methods of making proteins that are stable over an extended period of time. The invention specifically pertains to compositions and methods of making therapeutic protein formulations that remain stable at physiological temperature for extended release of therapeutic protein. In particular, the invention pertains to a biphasic pharmaceutical formulation.

**BACKGROUND**

The extended release delivery of a therapeutic protein toward a biologically relevant target is desirable for the treatment of medical conditions, such as cancer, cardiovascular disease, vascular conditions, orthopedic disorders, dental disorders, wounds, autoimmune disease, gastrointestinal disorders, and ocular diseases. Biocompatible and biodegradable polymers and other implantable delivery devices for the controlled and extended delivery of drugs have been in use for decades. For example, in some polymer-based delivery devices, as the polymer degrades over time, the therapeutic drug is slowly released.

Extended release can be desirable for patient compliance. In particular, reducing the number of injections can be beneficial, especially where a doctor is required to do the injection, such as in the case of intraocular therapeutics. There is an unmet medical need for extended release formulations to deliver drugs effectively over time with as few injections as possible. In the case of other diseases, for example cancer and diseases of inflammation, there is a need for improved implantable extended release formulations containing stable and effective protein therapeutics.

Therapeutic macromolecules, such as antibodies and receptor Fc-fusion proteins, must be formulated in a manner that not only makes the molecules suitable for administration to patients, but also maintains their stability during storage and while at the site of administration. For example, therapeutic proteins (*e.g.*, antibodies) in liquid solution are prone to degradation, aggregation and/or undesired chemical modifications unless the solution is formulated properly. The stability of a protein therapeutic in liquid formulation depends not only on the kinds of excipients used in the formulation, and the amounts and proportions of those excipients relative to one another, but also on the concentration of the soluble protein. Considerations aside from stability must also be taken into account when preparing a therapeutic protein formulation. Examples of such additional considerations include the viscosity of the solution and the concentration of antibody that can be accommodated by a given formulation. Thus, when formulating a therapeutic protein for extended release, great care must be taken to arrive at a formulation that remains stable over time and at storage and physiological temperature, contains an adequate concentration of antibody, and possesses other properties which enable the formulation to be conveniently administered to patients.

While small molecule formulations may use PEG, with larger proteins, PEG is known to cause precipitation. PEG is used to precipitate proteins to facilitate purifying, concentrating, and exchanging buffer. PEG is also used to crystalize protein for biophysical analysis. PEG is also used in small amounts (e.g., generally no more than 3% w/v) as an excipient to protect proteins against agitation stress. Precipitation of proteins by PEG is also known to aggregate proteins and to be deleterious to protein function.

The following documents are mentioned:

- Herrmann et al (2007) Pharmaceutical Research, 24(8): 1527-1537 which is concerned with new insight into the role of polyethylene glycol acting as protein release modified in lipidic implants;

- Atha et al (1981) Journal of Biological Chemistry, 256(23): 12108-12117 which is concerned with mechanism of precipitation of proteins by polyethylene glycol analysis in terms of excluded volume; and

- US 2012/095439 which is concerned with an implantable therapeutic device.

**SUMMARY**

In one aspect, the invention provides a biphasic pharmaceutical formulation comprising:

a. at least 25 mg/mL of polyethylene glycol (PEG);

b. at least 100 mg/mL of a VEGF antagonist, wherein less than 50% of the VEGF antagonist is present in a dissolved form in a liquid phase with PEG, and the remainder of the VEGF antagonist is present in a solid precipitate form in equilibrium with the dissolved form, wherein the solid precipitate form is excluded from the liquid phase by PEG; and;

c. a semi-permeable membrane, wherein the semi-permeable membrane surrounds the liquid phase and preferentially retains PEG while allowing diffusion of the VEGF antagonist through the membrane, wherein said VEGF antagonist has at least 80% native conformation, structure or function in the liquid phase for at least 30 days at physiological temperature, and said liquid phase has access to an environment with a lower concentration of said VEGF antagonist through the semi-permeable membrane.

In one embodiment, a semi-permeable membrane provides access of the protein to the environment.

In one aspect, the invention provides a drug delivery device for the extended delivery of a drug which remains stable throughout an extended period of time at physiological temperature. The drug delivery device of the invention comprises a stable biphasic pharmaceutical formulation of the present invention, a reservoir chamber containing the stable biphasic pharmaceutical formulation, a housing that encompasses the reservoir chamber, and a semi-permeable membrane in the housing that permits diffusion of the drug from the reservoir chamber to an external aqueous environment.

Also disclosed is a method of providing extended release for a pharmaceutical protein. According to this instance, a VEGF antagonist is combined with polyethylene glycol (PEG) to a final concentration of at least 100 mg/mL protein and at least 50 mg/mL PEG to form a biphasic mixture. In one instance, the VEGF antagonist is combined with PEG in the solid state, such that the final concentration of the VEGF antagonist represents the mass of the VEGF antagonist per total volume. The biphasic mixture contains the VEGF antagonist in an insoluble form and a soluble form. Less than 50% of the total amount of VEGF antagonist is in the soluble form along with an amount of PEG. According to this aspect, the soluble VEGF antagonist has access to an outside environment having a lower concentration of the VEGF antagonist. A semi-permeable membrane provides access of the VEGF antagonist to the outside environment. Over time, the VEGF antagonist diffuses through the semi-permeable membrane into the surrounding aqueous environment, and the overall concentration of VEGF antagonist in the biphasic mixture eventually declines to below 100 mg/mL However, the soluble phase will have a constant predetermined VEGF antagonist concentration until the insoluble phase is depleted and the concentration of VEGF antagonist in the soluble phase falls below the solubility limit. The predetermined VEGF antagonist concentration in the soluble phase is based on the solubility of the VEGF antagonist in the presence of PEG and the PEG concentration in the reservoir.

Also disclosed is a biphasic pharmaceutical formulation of the present invention for use in the treatment of a patient in need of the constituent drug. Also disclosed is a biphasic pharmaceutical formulation of the present invention for use in a method of treating a patient in need of the constituent drug by administering the biphasic pharmaceutical formulation to the patient. In one instance, the biphasic pharmaceutical formulation is contained in a reservoir device that is implanted into the patient. The biphasic pharmaceutical formulation contains at least 25 mg/mL of PEG and at least 100 mg/mL of the constituent drug, which is a VEGF antagonist. Less than 50% of the drug is in the soluble phase along with an amount of PEG. The remainder of the drug is in the insoluble phase. The soluble drug has access through a semi-permeable membrane of other porous structure to an environment having a lower concentration of drug (*e.g.*, patient tissue or aqueous fluid outside of the devices) and diffuses to its target in the patient. In some embodiments of the biphasic pharmaceutical formulation contained in a reservoir device, the soluble drug has access to the environment through a port, such as a semi-permeable membrane. The drug, according to this aspect, is stable for at least 30 days under physiological conditions, and as the drug diffuses from the biphasic pharmaceutical formulation, the amount of drug in the formulation declines below its initial concentration.

**DRAWINGS**

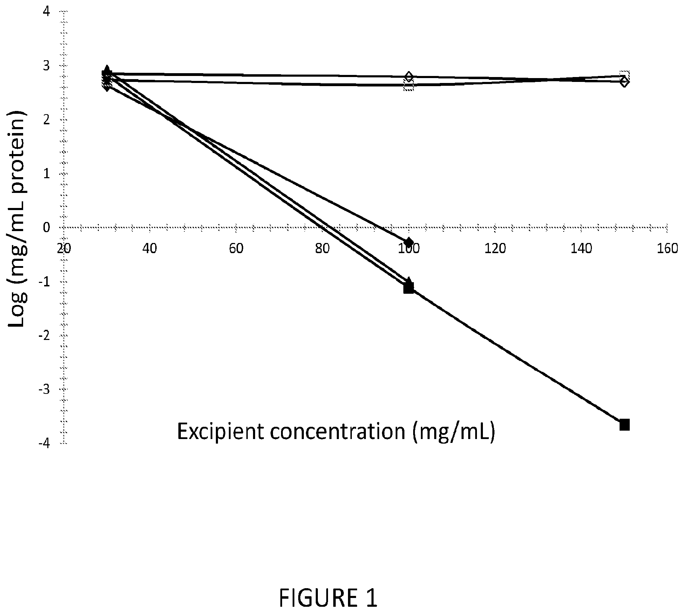

Figure 1 is a line graph depicting the effect of various excipients on soluble protein concentration. The X-axis depicts excipient concentration in milligrams per milliliter (mg/mL). Open squares (□) represent dextran 6000 excipient; open diamonds (◊) represent dextran 40000 excipient; closed diamonds (♦) represent polyethylene glycol 3,350 MW (PEG3350) excipient; closed squares (■) represent PEG8000 excipient; and closed triangles (▲) represent PEG20000 excipient. The Y-axis depicts the logarithm of protein concentration in mg/mL.

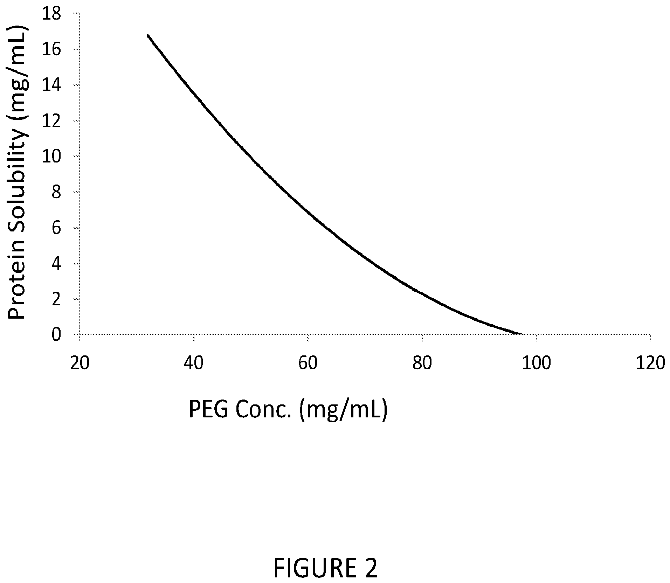

Figure 2 is a line graph depicting the effect of polyethylene glycol concentration in mg/mL (X-axis) on protein solubility expressed in mg/mL (Y-axis).

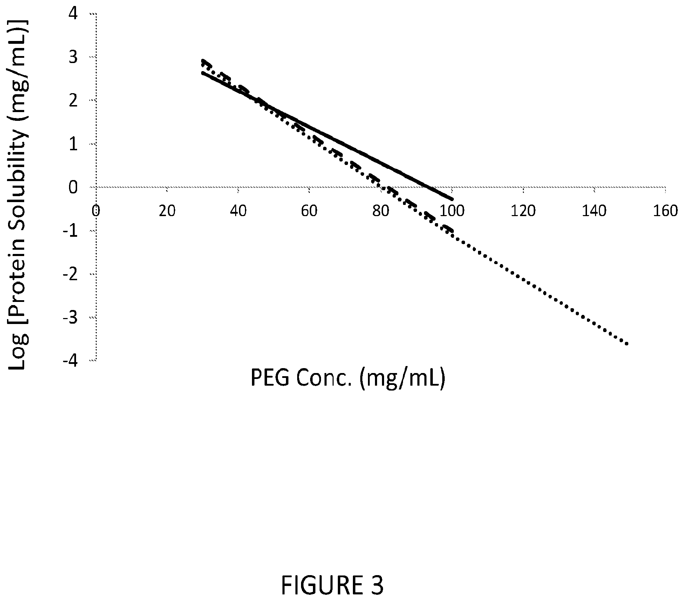

Figure 3 is a line graph depicting the effect of various molecular weight forms of PEG excipient on soluble protein concentration. The X-axis depicts excipient concentration in milligrams per milliliter (mg/mL). The solid line represents PEG3350 excipient; the dotted line represents PEG8000 excipient; and the dashed line represents PEG20000 excipient. The Y-axis depicts the logarithm of protein concentration in mg/mL.

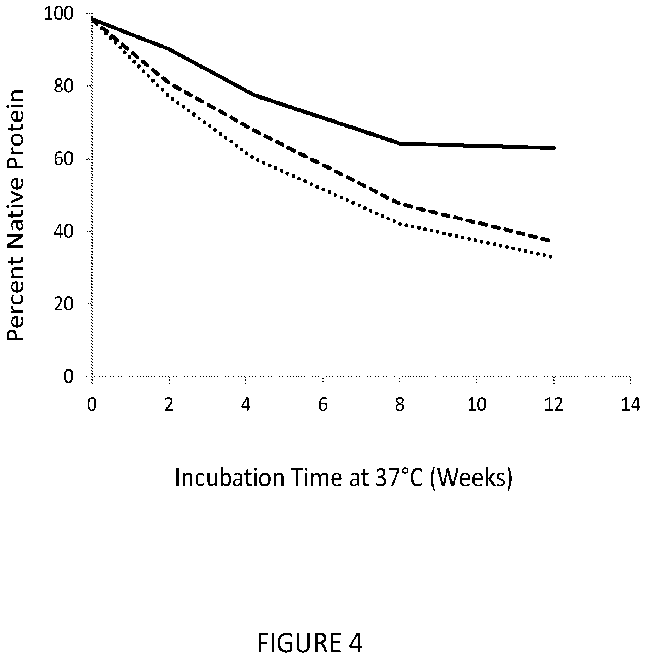

Figure 4 is a line graph depicting the stability of biphasic protein at 4.5 mg/mL aqueous concentration (30 mg/ml total concentration) in the presence of 80 mg/mL of different molecular weight forms of PEG as a function of incubation time at 37°C in phosphate-buffered saline (pH 7.2). The solid line represents PEG3350 excipient; the dashed line represents PEG8000 excipient; and the dotted line represents PEG20000 excipient. The X-axis depicts incubation time in weeks. The Y-axis depicts the relative percentage of protein having native conformation (expected hydrodynamic radius).

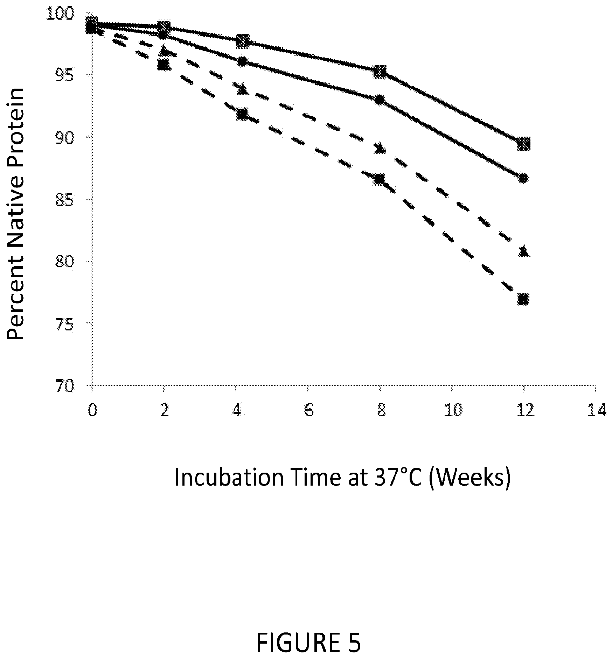

Figure 5 is a line graph depicting the stability of soluble protein in the presence of different amounts of PEG3350 as a function of incubation time at 37°C. The solid line and filled square (■) represents a control with 4.5 mg/mL protein and no PEG; the solid line and filled circle (•) represents a control with 10 mg/mL protein and no PEG; the dashed line and filled triangle (▲) represents 80 mg/mL PEG3350 and 4.5 mg/mL soluble protein; and the dashed line and filled square (■) represents 65 mg/mL PEG3350 and 10 mg/mL soluble protein. The X-axis depicts incubation time in weeks. The Y-axis depicts the relative percentage of protein having native conformation (expected hydrodynamic radius).

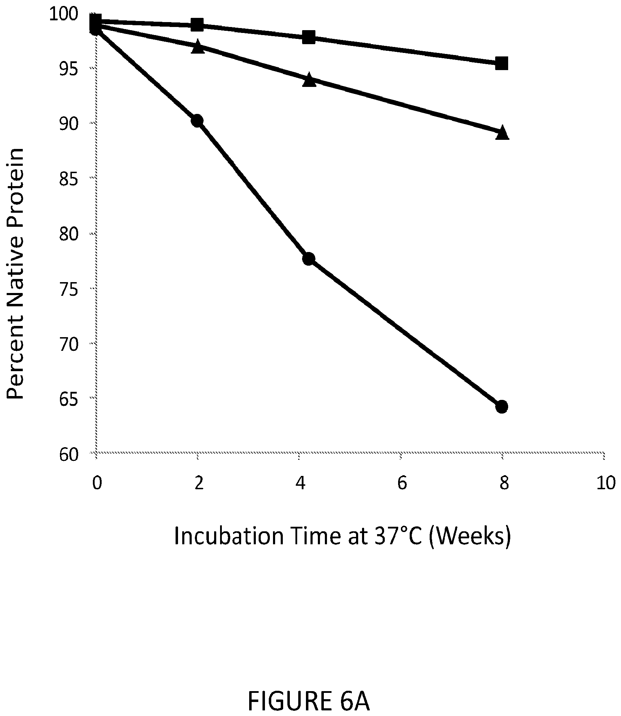

Figure 6A is a line graph depicting the stability of monophasic soluble protein and biphasic soluble protein in the presence of PEG3350 as a function of incubation time at 37°C. The data points with a filled square (■) represent a control sample with 4.5 mg/mL protein and no PEG; the data points with a filled triangle (▲) represent monophasic soluble protein at a concentration in of 4.5 mg/mL and PEG3350 at about 80 mg/mL; the data points with a filled circle (•) represent biphasic soluble protein at a concentration of 4.5 mg/mL, PEG3350 at about 80 mg/mL, and 25.5 mg/mL biphasic insoluble protein. The X-axis depicts incubation time in weeks. The Y-axis depicts the relative percentage of protein having native conformation (expected hydrodynamic radius).

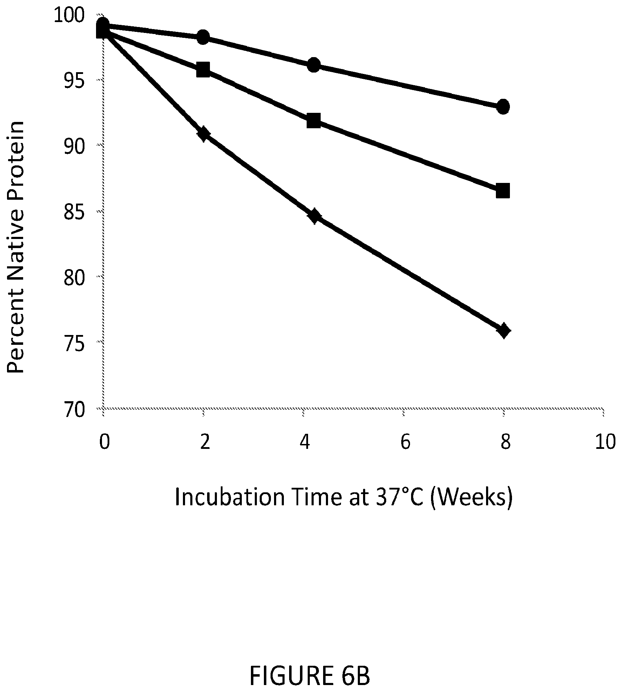

Figure 6B is a line graph depicting the stability of monophasic soluble protein and biphasic soluble protein in the presence of PEG3350 as a function of incubation time at 37°C. The data points with a filled circle (•) represent a control sample with 10 mg/mL protein and no PEG; the data points with a filled square (■) represent monophasic soluble protein at a concentration in of 10 mg/mL and PEG3350 at about 65 mg/mL; the data points with a filled diamond (♦) represent biphasic soluble protein at a concentration in of 10 mg/mL, PEG3350 at about 65 mg/mL, and 20 mg/mL biphasic insoluble protein. The X-axis depicts incubation time in weeks. The Y-axis depicts the relative percentage of protein having native conformation (expected hydrodynamic radius).

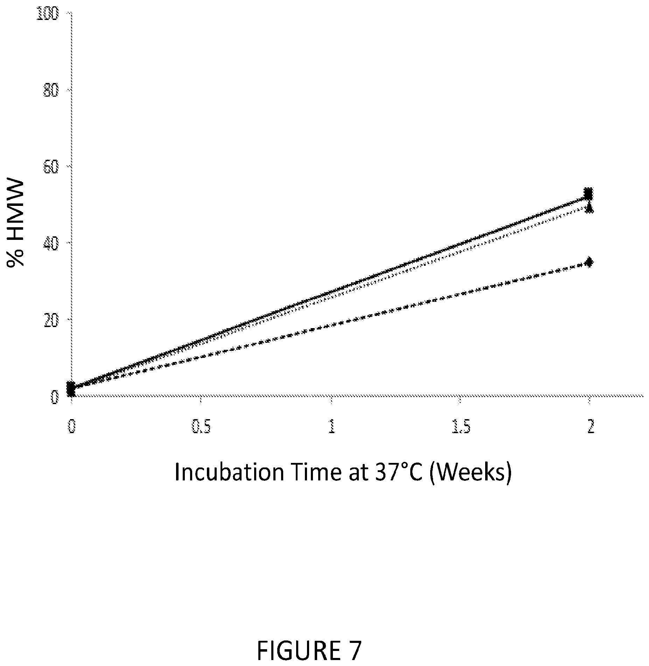

Figure 7 is a line graph depicting the stability of insoluble protein in the presence of 150 mg/ml PEG3350 as a function of incubation time at 37°C. 30 mg/mL of insoluble protein in the presence of 150 mg/mL PEG3350 is represented by the short dashed line with closed diamonds (♦). 60 mg/mL of insoluble protein in the presence of 150 mg/mL PEG3350 is represented by the solid line with closed squares (■). 120 mg/mL of insoluble protein in the presence of 150 mg/mL PEG3350 is represented by the dotted line with closed triangles (▲). The X-axis depicts incubation time in weeks. The Y-axis depicts the percentage of protein that is of a high molecular weight relative to total protein. After 2 weeks incubation at 37°C, insoluble protein was not fully recoverable and accurate levels of aggregate could not be determined.

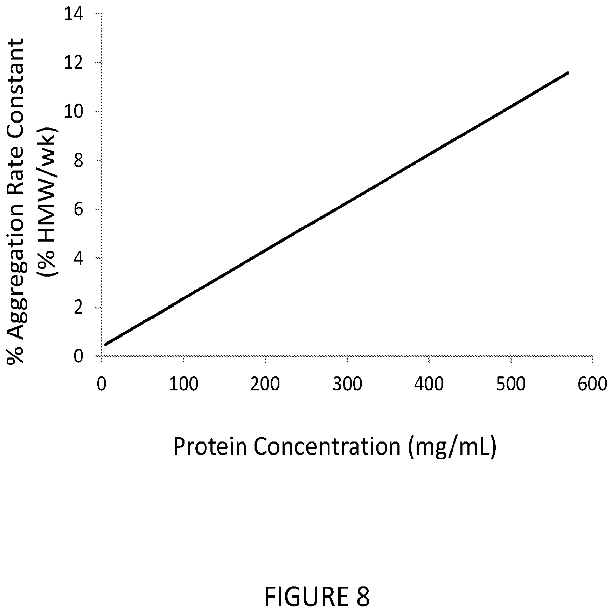

Figure 8 is a line graph depicting the effect and projected effect of protein concentration on the rate of protein aggregation and the formation of high molecular weight species. The X-axis depicts protein concentration in mg/mL. The Y-axis depicts the percent change in high molecular weight species formation per week.

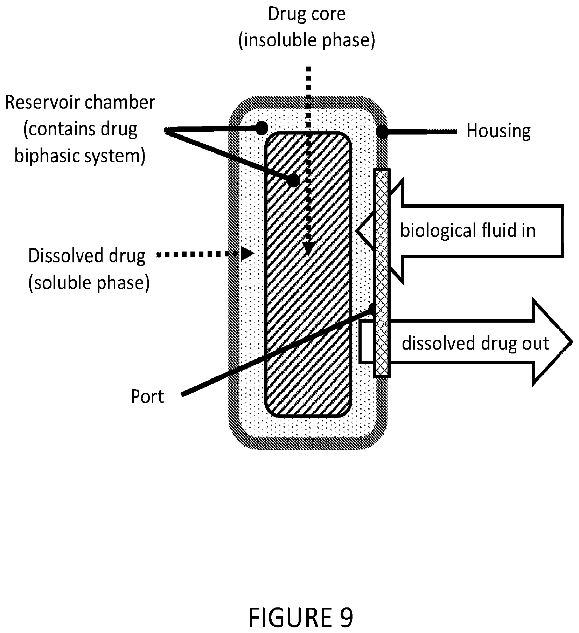

Figure 9 depicts a schematic of a reservoir device comprising an outer housing surrounding (encompassing) a reservoir chamber containing a biphasic formulation. The housing contains a port to allow egress of drug from the reservoir.

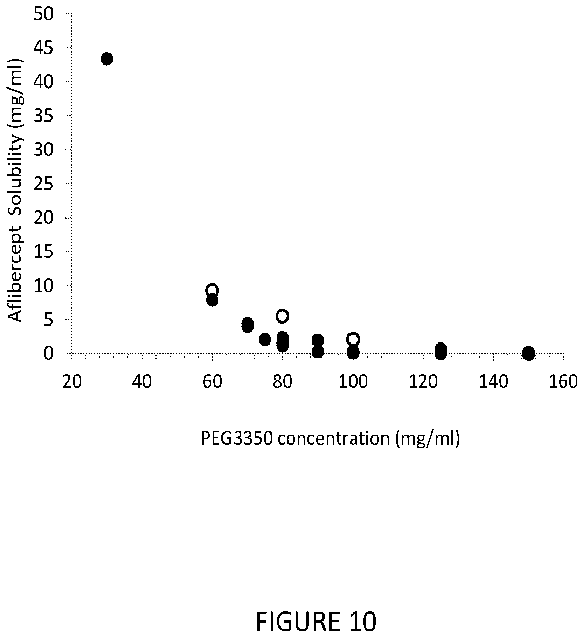

Figure 10 depicts the solubility of aflibercept (VEGF trap) expressed in mg/ml as a function of PEG 3350 concentration. Open circles (○) represent the solubility of reconstituted solid (dry) aflibercept (including sucrose) plus dry PEG subsequently hydrated. Closed circles (•) represent the solubility of liquid formulation of PEG and aflibercept without sucrose.

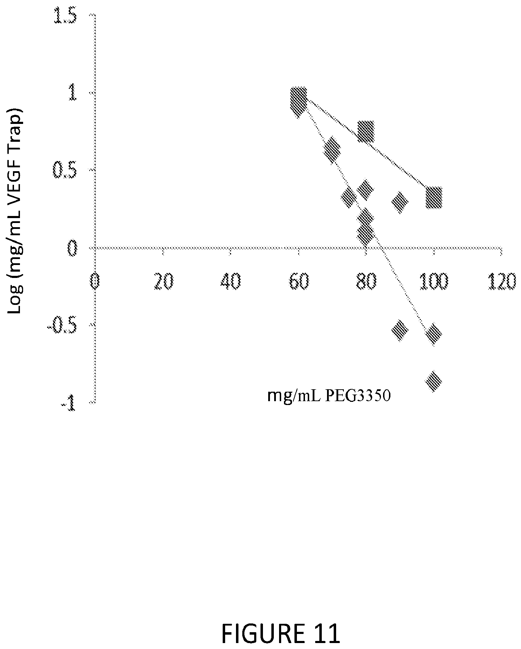

Figure 11 depicts the solubility of aflibercept expressed in log (mg/ml) as a function of PEG 3350 concentration. Squares (□) represent the combination of dry aflibercept (including sucrose) plus dry PEG subsequently hydrated. Diamonds (◊) represent the combination of PEG solution and aflibercept solution without sucrose.

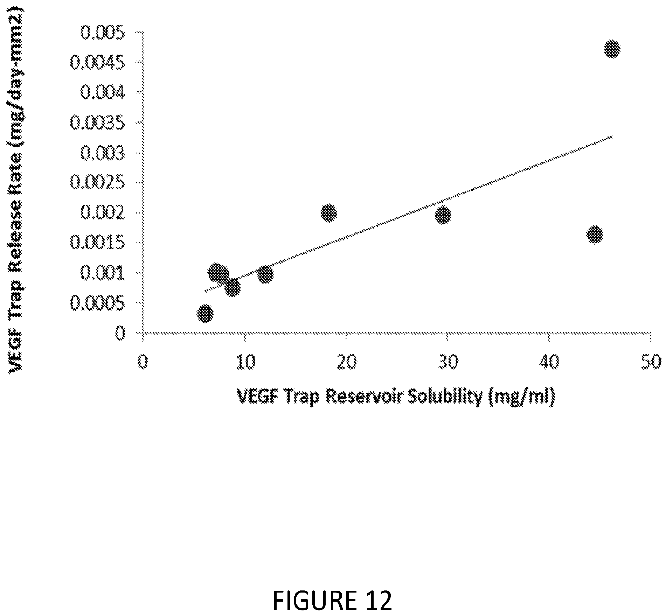

Figure 12 depicts the release rate of aflibercept expressed in milligrams released per day per square millimeter of porous structure surface area as a function of concentration of soluble aflibercept.

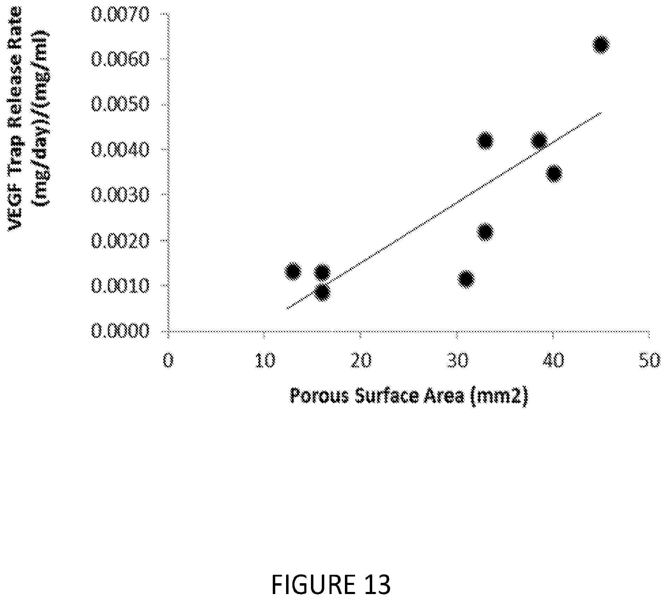

Figure 13 depicts the release rate of aflibercept expressed in milligrams released per day per mg/ml of soluble aflibercept as a function of surface area of porous structure.

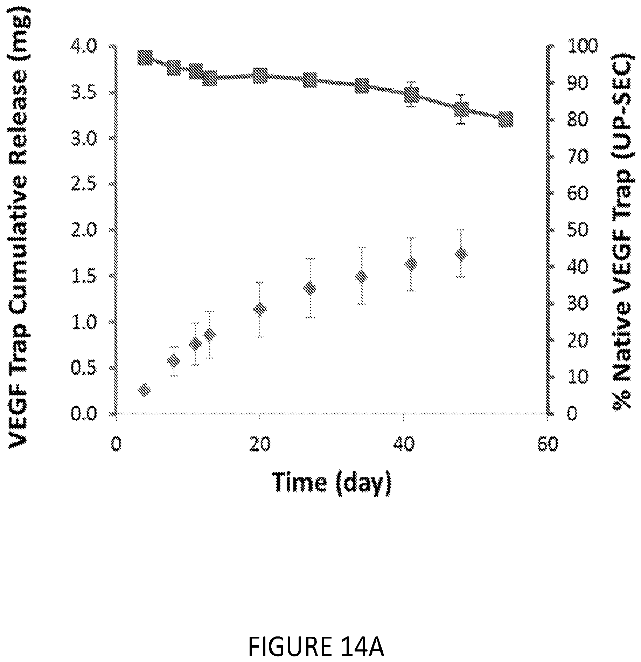

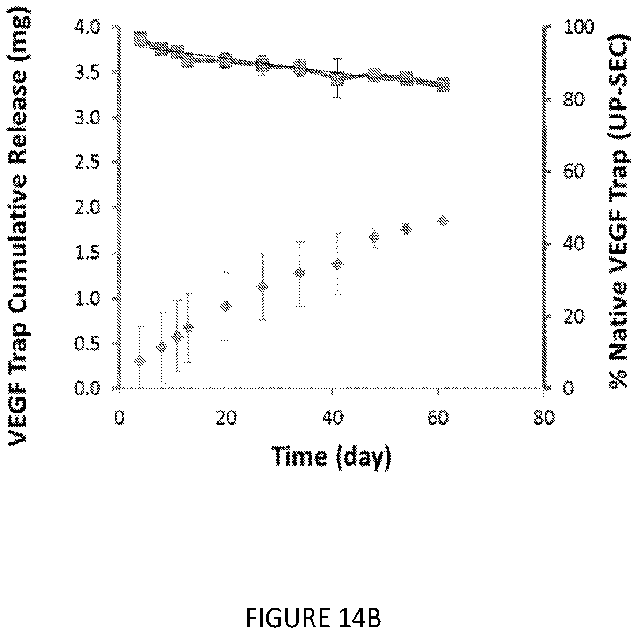

Figure 14 depicts the cumulative release of aflibercept expressed in milligrams (diamonds [◊]), and the stability of aflibercept expressed as percent native conformation as determined by size-exclusion chromatography (squares [□]) as a function of time at physiological temperature and pH. Panel A depicts the results for 100 ($\pm$ 3) mg/ml PEG 3350 (n=2). Panel B depicts the results for 70 ($\pm$ 3) mg/ml PEG 3350 (n=4).

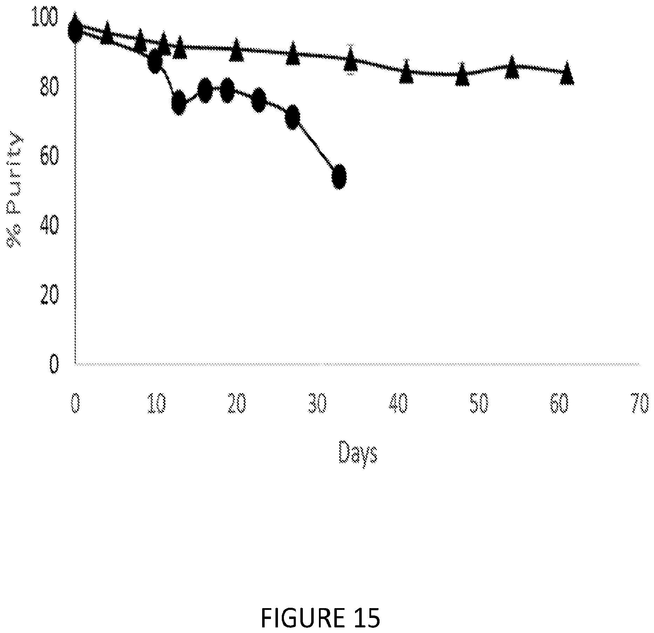

Figure 15 depicts the stability of released aflibercept as a function of time. The X-axis depicts time in number of days at physiological temperature. The Y-axis depicts the relative amount of native aflibercept remaining in the sample (percent purity). Closed triangles (▲) represent aflibercept released from a formulation containing 80 mg/mL $\pm$ 20 mg/mL PEG3350. Closed circles (•) represent aflibercept released from a formulation containing sucrose only (no PEG).

## DETAILED DESCRIPTION

### Biphasic System

Formulating protein biotherapeutics at a high concentration creates significant challenges for the biopharmaceutical industry. Proteins at high concentration (e.g., > 50 mg/mL) tend to be unstable and prone to increased rates of aggregation. This is especially problematic for extended release formulations where high levels of drug are required. Since extended release formulations are designed to release drug over long periods of time in physiological environments, the drug must be stable and biologically active during that extended period.

The present invention includes combining polyethylene glycol (PEG) and a protein to provide a stable protein in a solid precipitate form in equilibrium with a liquid saturated soluble form of the protein, in which the soluble phase has access to an environment with a lower concentration of the protein, *i.e.* the system is open, not closed. While not being limited by any mechanism, in theory, the protein is stable in both phases and the protein in the insoluble phase feeds the soluble phase which in turn drives diffusion of the protein through the semi-permeable membrane. Protein stability refers to the protein that diffuses out of the reservoir. In an aspect, the open biphasic system allows for a semi-permeable membrane or other porous structure to preferentially retain PEG while allowing diffusion of protein.

An example of an open system is a device with porous walls that allows the soluble form of the protein to have access to the outside environment and allows the soluble form of the protein to be released over time. In some embodiments, the soluble phase has access to environment through a port, in particular a semi-permeable. The semi-permeable membrane of the subject invention allows the free passage of water and protein through it. In some embodiments, the semi-permeable membrane comprises nanopores and/or micropores. In some embodiments, the semi-permeable membrane is a nanoporous and/or microporous polymer film.

The final concentration of the PEG is 25 mg/mL or more. The concentration of the VEGF antagonist in undissolved and dissolved form is 100 mg/mL or more. In some embodiments, the VEGF antagonist is combined with the PEG in a solid form, such as a lyophilized protein or spray-dried protein.

The present invention includes a biphasic system for the long term delivery of a stable drug. By biphasic, what is meant is that the drug exists in in two phases: an insoluble (precipitated) or solid phase, and a soluble or liquid phase. The insoluble phase serves as a drug reservoir, and the soluble phase serves as the active form of the drug that diffuses into a physiological system and contacts the physiological target. In a closed biphasic system, as in a vial, the soluble phase is a saturated solution of the drug in equilibrium with the precipitate. In an open biphasic system, however, as the soluble drug that has access to the outside aqueous environment diffuses out from the saturated soluble phase, a portion of the precipitated drug is solubilized and enters the saturated soluble phase to replace the soluble drug that has diffused into the environment. The protein in the precipitated phase is slowly solubilized to maintain equilibrium with the saturated phase, i.e., the precipitated phase replenishes the soluble phase, and the soluble phase diffuses into the physiological system to engage the drug target (Equation 1). By controlling the concentration of the precipitator excipient (*i.e.*, PEG), the soluble concentration of drug in the saturated soluble phase is controlled.

$$\text{Eq. 1:} \quad \underset{\text{(Insoluble Phase)}}{\text{Precipitate}} \rightleftarrows \underset{\text{(Soluble Phase)}}{\text{Saturated Solution}} \longrightarrow \underset{\text{(Diluted Phase)}}{\text{Physiological Environment}}$$

In one embodiment, the biphasic system is for use in a method where it is implanted into a target physiological environment. For example, the biphasic system can be delivered into the vitreous humor in those cases where the drug target tissue is the retina, macula, or other internal eye structure. For other targets, the system can be implanted subcutaneously, subdermally, or intraperitoneally.

The biphasic system is partially enclosed within a barrier. By partially enclosed, what is meant is that the barrier allows diffusion of solubilized drug from the biphasic system. The barrier allows drug to diffuse out of the biphasic system and contact the target, but prevents the precipitator excipient from rapidly diffusing out. The barrier can be designed and tuned to allow specific rates of release of drug. For example, the barrier may be a membrane containing pores that permit the passage of the drug. The number and/or size or architecture of the pores as well as the overall area of the semi-permeable membrane can be designed to regulate the release rate of the drug.

The precipitator excipient may be selected from known protein precipitators based upon the specifications of the drug elution device. Common precipitators include lyotropic salts, such as ammonium sulfate, miscible solvents like acetone, and non-ionic macromolecules like dextran and PEG. The selection of the precipitator, as in the case of the enclosure barrier, depends upon the solubility of the drug and the desired concentration of the drug in the soluble phase. For example, dextrans, while reportedly used to precipitate proteins, do not limit solubility of proteins in the same range of excipient concentration as PEG. Figure 1 depicts the comparison of the effect of PEG 3350 (closed diamonds [♦]), PEG 8000 (closed squares [■]), PEG 20000 (closed triangle [▲]), dextran 6000 (open square [□]) and dextran 40000 (open diamond [◊]) on protein solubility. Note that both molecular weight forms of dextran leave more protein in the soluble phase than does PEG at similar excipient concentrations. Higher concentrations of dextran are needed to attain the same level of protein precipitation as lower concentrations of PEG. Hence, a biphasic pharmaceutical formulation of the present invention comprises at least 25 mg/ml of PEG. In one embodiment, PEG is preferable to form a biphasic formulation with a large reservoir for extended release of smaller doses of drug over a longer period of time.

In one embodiment, PEG is selected as the precipitator excipient for a long term implantable open biphasic therapeutic protein formulation. The concentration of protein within the soluble phase of the biphasic system can be controlled by adjusting the concentration of PEG. For example, combining about 16 mg/mL of a 150 kDa protein with about 25 mg/mL to about 150 mg/mL of PEG provides a range of concentration of the protein in the soluble fraction of about 14 mg/mL to less than 1 mg/mL (Figure 2). This effect of PEG on protein solubility depends upon the mass concentration of the PEG, and not on the molar concentration of PEG. In other words, the same effect on protein solubility (i.e., the concentration of protein in the saturated soluble phase) was observed for the same amounts by mass of PEG 3350, PEG 8000, and PEG 20000 (Figure 3). That is 80 mg/mL of PEG 20000 has the same effect as 80 mg/mL of PEG 3350 on the same protein.

While the concentration of drug in the soluble phase depends on excipient concentration and is independent of overall drug content, the amount of drug in the insoluble phase includes all drug that is in excess of drug in saturated soluble phase. Therefore, the amount of drug in the insoluble phase depends on both PEG concentration and total amount or final concentration of drug.

PEG is expected to impact not only protein solubility, but also protein stability. While the particular molecular weight species of PEG has very little or no impact on the stability of fully dissolved protein in a monophasic system, it does have an effect on the stability of fully dissolved protein in a biphasic system. Lower molecular weight PEG, e.g., PEG 3350, has less of a negative impact on the stability of a 100 kDa -150 kDa protein in the soluble phase of a biphasic system than higher molecular weight species of PEG, e.g., PEG 8000 or PEG 20000 (Figure 4).

Even though PEG 3350 has less of a negative impact on protein stability in the soluble phase than higher molecular weight forms, it still destabilizes protein in a closed system. As shown in Figure 5, protein stability is decreased in the presence of PEG3350 relative to no PEG in a fully dissolved monophasic system. The protein destabilizing effect of PEG is more pronounced in the closed biphasic system (Figures 6A and 6B), indicating that protein stability is decreased when kept in the insoluble phase rather than in the dissolved phase in a closed system, but at the concentrations shown, there is no benefit in either the fully dissolved or the biphasic system.

When PEG concentration is increased in a closed system, the stability of protein in the insoluble phase decreases as measured by an increase in aggregation rate (See Figures 4-7). When PEG is present in concentrations that limit protein solubility to less than 0.1 mg/ml, irreversible protein precipitation is observed. In an aspect of the present invention, aggregation in the insoluble phase of the system is greater than in the solution phase in the closed system.

A high protein concentration in the soluble phase of a closed system reduces protein stability in both the PEG-free and PEG-inclusive formulations (Figures 6A and 6B). For example, with sucrose only (no PEG) in a monophasic system under uniform conditions, a 100 kDa to 150 kDa protein at about 4.5 mg/mL has an aggregation rate of about 0.35% HMW/wk, an aggregation rate of about 2.7% HMW/wk at about 120 mg/mL, and an extrapolated aggregation rate of about 10.2% HMW/wk at a projected 500 mg/mL concentration (Figure 8). This high rate of aggregation is unacceptable for pharmaceutical applications of high concentration protein formulations.

In contrast to a closed system, the addition of PEG improves the stability of highly concentrated proteins in an open biphasic system. For example, the aggregation rate of saturated soluble protein in a closed monophasic system at 10 mg/mL containing 65 mg/mL PEG can be about 1.3 % HMW/wk. In a closed biphasic system containing 10 mg/mL soluble phase protein and 20 mg/mL insoluble phase protein containing 65 mg/mL PEG, the aggregation rate can be about 2.5 % HMW/wk, in a closed biphasic system containing 10 mg/ml soluble phase protein and 110 mg/ml insoluble phase protein containing 65 mg/ml PEG, the aggregation rate can be about 10% HMW/wk, but in an open biphasic system containing 75 mg/ml PEG and >100 mg/ml total protein, the aggregation rate can be about 0.8 % HMW/wk.

In a closed system such as in a vial, the protein is not released and the protein mass is not transferred from the insoluble phase to the soluble phase. In an open system, e.g., where the biphasic system is implanted in a physiological system, the concentration of protein in the insoluble phase (*i.e.*, the total mass of protein in the precipitate) decreases as protein is transferred from that phase to the saturated soluble phase as protein from the saturated phase diffuses into the physiological environment. Given the continual shift of protein from the insoluble phase to the soluble phase, the protein in a dynamic open biphasic system, where soluble protein moves out of the biphasic reservoir, is more stable than the closed system or a monophasic solution.

In addition to the amount of PEG and protein that are combined to make the biphasic system, the effect of PEG on protein solubility and phase partitioning depends in part on specific protein attributes, such as for example protein size and/or isoelectric point. Table 1 depicts the projected or actual effect of various amounts of PEG on the solubility of a receptor Fc-fusion protein (trap), immunoglobulin protein (IgG), human serum albumin (HSA), and fibrinogen. Atha et al., "Mechanism of Precipitation of Proteins by Polyethylene Glycols," J. of Bio. Chem. 256(23):12108-12117 (1981) is incorporated herein by reference for its description of the effect of PEG on the solubility of IgG#2, HSA and fibrinogen.

TABLE 1

| Protein | % w/v PEG | Protein solubility (mg/mL) | % w/v PEG | Protein solubility (mg/mL) |
|---|---|---|---|---|
| Trap | 3.0 | 47 | 10 | 1.6 |
| IgG#1 | 3.0 | 98 | 12 | 0.2 |
| IgG#2 | 8.0 | 8 | 18 | 0.1 |
| HSA | 28 | 11 | 35 | 0.8 |
| Fibrinogen | 2.5 | 10 | 7.5 | 0.1 |

## Reservoir Device

In some embodiments, an open biphasic system for the delivery of a VEGF antagonist to a patient is housed within a reservoir device. A reservoir device of the present invention is partially open to the environment and the rate of diffusion or movement of the soluble phase drug from the biphasic system can be tuned. In some embodiments, a biphasic drug-charged reservoir device system is for use in a method where it is implanted into a patient such as by subcutaneous delivery. A device of the present invention can come into contact with a physiological microenvironment and the solubilized drug emerges from the device and diffuses into the physiological microenvironment at a pre-determined rate. In addition to the concentration of the protein in solution, this release rate is determined by properties of a device that can be adjusted to change the delivery rate. Those adjustable properties are size, structure of the reservoir device, porosity, filter size, etc. See Drug Delivery: Engineering Principles for Drug Therapy by W. Mark Saltzman, in Topics in Chemical Engineering, 1st Edition, Oxford University Press, New York, 2001; Int'l Patent Application Publication No. WO2008109886A1, Desai et al., published September 12, 2008; Int'l Patent Application Publication No. WO2012142318A1, Desai et al., published October 18, 2012; US Patent Application Publication No. 20140170204A1, Desai et al., published June 6, 2014; and US Patent Application Publication No. US20150216829A1, Desai et al., published August 6, 2015, which are hereby incorporated by reference for description of delivery rates and device properties to achieve them.

The delivery microenvironment influences the size, shape and constituent materials of the reservoir device. For example, a reservoir device for delivery to the intima may be sized and shaped to fit within the walls of a stent to be delivered through a catheter. The biphasic drug-charged device may be delivered to the patient topically - *e.g.,* a dermal patch-type device, enterally - *e.g.,* a capsule or suppository, or parenterally - *e.g.,* an implanted device. Exemplar microenvironments include the gastrointestinal system, the central nervous system (*e.g.*, epidural, intracerebral, intracerebroventricular, intrathecal), the amniotic-fetus system (*e.g.*, extra-amniotic), the macula, retina or other eye structures (*e.g.*, intravitreal, intraocular), the venous-arterial system (*e.g.*, intra-arterial), the bone and joint system (*e.g.*, intraosseous, intraarticular), muscular, subcutaneous, dermal, peritoneal, mucosal and the like.

In one embodiment of the invention, the reservoir device comprises a "chamber" ("reservoir chamber") and a "housing" that encompasses the chamber. The housing comprises one or more openings, ports, windows, pores, porous structures or permeable or semi-permeable material (collectively called "porous structure"). The "porous structure" permits the passage of drug out of the reservoir device, and in some embodiments the ingress of physiological solvent and solutes. In some embodiments, the entire housing comprises porous structure. In other embodiments, the porous structure comprises only part of the housing to serve as a window-like structure in an otherwise impermeable housing. The drug biphasic system is held within the reservoir chamber. In some embodiments, the insoluble phase provides a drug core and the soluble phase provides a drug cortex. Figure 9 depicts a schematic of an exemplar reservoir device.

The reservoir device of the present invention may be made of any material and be of any size to accommodate a specific target. For example, the device may in some cases be worn outside of the body, such as an insulin pump, and may therefore be relatively large. The device may be for use in a method where it is implanted into the body, *e.g.*, subcutaneous or intravitreal placement, which necessitates the device being small. For example, an intravitreal device may have a volume on the order of 1 to 50 μl.

In an embodiment of the present invention, a reservoir device that is for use is in a method where it is implanted into a physiological system may be made of biocompatible materials, examples of which include metals, including silicon, titanium, stainless steel, and nickel-titanium alloys, and polymers, including polylactic acid (PLA), polyglutamic acid (PGA), polycaprolactone (PCL), polyorthoester, polyanhydride, polyhydroxybutyrate, polyhydroxyvalerate, poly(amino acid), collagen, cellulose acetate, nylon, polycarbonate, poly(tetrafluoroethylene) (PTFE), polyether sulfone, polyvinylidene fluoride (PVDF), acrylates, polyacrylate, methlymethacrylate, polymethylmethacrylate (PMMA), polycyanoacrylate, siloxanes, polycarbonate, polyetheretherketone (PEEK), polyethylene, polyethylene terephthalate (PET), polyimide, polyamideimide, polypropylene, polysulfone, polyurethane, and polyvinylidene.

A reservoir device of the present invention may be rigid or flexible, single-use or refillable. U.S. Patent Application Numbers 2015/0250647, 2011/046870, 2013/0324942 and U.S. Patent Numbers 9,033,911 and 8,623,395 describe a rigid and refillable biocompatible intravitreal reservoir device for the delivery of an ophthalmic drug to the eye (*e.g.*, the FORSIGHT™ Vision4 device). In one embodiment, a device of the present invention is made from titanium and is refillable. As a refillable device, it can contain a penetrable barrier situated at or near the proximal end of the reservoir device, through which drug is deposited *via* a cannula. In such an embodiment, the porous structure comprises sintered metal.

The housing material of flexible devices of the present invention may be a polymer, and the semi-permeable membrane or other porous structure may be a polymeric film containing micropores, nanopores or other channels. Flexible and single-use polymer devices and methods of making them are disclosed in Bernards (2012), U.S. Patent Application Publication Nos. 2014/0170204 and 2015/0119807, and EP Patent Application No. EP2164425A1. US 2015/0119807 A1 describe thin film sandwich structures comprising nanoporous polycaprolactone (PCL) film, or a combination of microporous PCL and a non-porous backing that can be used in a device of the present invention. In such an embodiment, the drug substance is loaded between two polymer film layers, which are then heat sealed. The size and architecture of the pores, as well as the surface area of the semi-permeable membrane or other porous structure controls the rate of elution of the drug from the device. Furled drug-loaded devices can be injected through a needle cannula for example into the vitreous.

The term "nanopore" describes the general diameter of the pore or channel opening in a barrier that allows access of material on one side of the pore (*e.g.*, internal environment) to material on the other side of the pore (*e.g.*, outside or external environment). Pores having a diameter of about 100 nm or below are generally considered to be nanopores.

A nanopore of the present reservoir device has a diameter of between about 0.2 nm to about 100 nm. In some embodiments, the nanopore has a diameter of between about 0.2 nm and about 2 nm, between about 2 nm and about 50 nm, or between about 50 nm and about 100 nm. In some embodiments, the nanopore of the present reservoir device has a diameter of about 10 nm, about 11 nm, about 12 nm, about 13 nm, about 14 nm, about 15 nm, about 16 nm, about 17 nm, about 18 nm, about 19 nm, about 20 nm, about 21 nm, about 22 nm, about 23 nm, about 24 nm, about 25 nm, about 26 nm, about 27 nm, about 28 nm, about 29 nm, or about 30 nm.

A microporous film of the present invention contains micropores. A micropore of the present invention has a cross-section diameter of about 1 micron to about 2 microns, about 2 microns to about 5 microns, about 1 micron, about 1.1 microns, about 1.2 microns, about 1.3 microns, about 1.4 microns, about 1.5 microns, about 1.6 microns, about 1.7 microns, about 1.8 microns, about 1.9 microns, or about 2 microns. In some embodiments, the micropore has a diameter of less than about 10 microns. In some embodiments, the micropore has an interconnecting pore size of less than about 1 micron.

The reservoir device of the present invention can be an implantable, self-regulating mechanochemical pump. Such a device uses flexible membranes embedded with enzymes that respond to changes in the microenvironment. Those changes cause the membrane to swell and consequently open its pores to allow access of the soluble protein to the outside environment and consequent efflux of soluble drug. For example, a cross-linked hydrogel membrane may be designed to swell when amine groups of polymer side chains are protonated, enabling the device to open in response to a pH change in the microenvironment. The drug is subsequently "pumped" from the reservoir chamber and into the physiological system. Enzymes that reduce or oxidize a particular chemical may be embedded into the membrane to serve as the source or sink of protons. For examples of mechanochemical devices, see Kost, et al. J. Biomed. Mater. Res. 19, 1117-1133 (1985), Albin et al., J. Controlled Release 6, 267-291 (1987)], Ishihara et al., Polymer J. 16, 625-631 (1984), and De Juan et al., U.S. Patent No. 9,033,911, May 19, 2015.

**Definitions**

The invention includes a formulation for improved solubility of a protein over an extended period of time relative to a monophasic solution. In another aspect, the invention provides a drug delivery device for the extended delivery of a drug which remains stable throughout an extended period of time at physiological temperature. Also provided is a method of making said drug formulation for such a delivery device, and (iii) said formulation or device for use in a method to treat a patient in need of the constituent drug. A drug formulation of the present invention includes a biphasic system containing a "first molecule" that exists in a soluble phase and insoluble phase, and a "second molecule", which is an excipient that reduces the effective solubility of the first molecule. In some embodiments, the biphasic system is contained within a reservoir chamber, which may be bounded by a housing to form a reservoir device. The "first molecule" is generally a biological molecule having therapeutic utility or other drug or drug substance.

The term "biphasic" refers to the status of a molecule coexisting in a solid precipitated phase and in a saturated soluble phase. A biphasic mixture of molecules is in equilibrium. In a biphasic mixture in a closed system (*e.g.*, as in a vial or other container not in contact with a physiological system), the overall amount of the molecule in each phase remains relatively constant. In a biphasic mixture in an open system, where the soluble form of the molecule can diffuse away from the biphasic mixture (*e.g.*, when implanted or otherwise in communication with a physiological or other system), the amount of the molecule in the biphasic system will decline over time. In the open system, the insoluble fraction of the molecule serves as a reserve form of the molecule. For example, a precipitated protein surrounded by a saturated solution of the same protein is a "biphasic system" and the protein is "biphasic".

The term "biomolecule" denotes a biological molecule, which includes macromolecules such as proteins, carbohydrates, lipids, nucleic acids, and synthetic composite molecules such as aptamers, as well as small molecules such as primary metabolites, secondary metabolites, and products produced in biological systems. Biological molecules include *inter alia* proteins, polypeptides, peptides, and amino acids, aptamers, lipids, nucleosides, nucleotides, and nucleic acids, and carbohydrates. Biomolecules may be naturally occurring, *i.e.,* produced in naturally occurring organisms, viruses and environments, or artificial, *i.e.,* produced by synthetic chemical means or in biological systems containing recombinant or ectopic nucleic acids. In some specific embodiments, the biomolecule is a therapeutic biomolecule. A therapeutic biomolecule is a "drug" or "drug substance". In some embodiments, the "first molecule" of the device is a protein.

The term "protein" means any amino acid polymer having more than about 50 amino acids covalently linked via amide bonds. Proteins contain one or more amino acid polymer chains, generally known in the art as "polypeptides". A protein may contain one or multiple polypeptides to form a single functioning biomolecule. "Polypeptides" generally contain over 50 amino acids, whereas "peptides" generally contain 50 amino acids or less.

As used herein, "protein" can include any of biotherapeutic proteins, recombinant proteins used in research or therapy, trap proteins and other chimeric receptor Fc-fusion proteins, chimeric proteins, antibodies, monoclonal antibodies, polyclonal antibodies, human antibodies, and bispecific antibodies. In another aspect, a protein can include antibody fragments, nanobodies, recombinant antibody chimeras, cytokines, chemokines, peptide hormones, and the like. Proteins may be produced using recombinant cell-based production systems, such as the insect bacculovirus system, yeast systems (*e.g.*, *Pichia* sp.), mammalian systems (*e.g.*, CHO cells and CHO derivatives like CHO-K1 cells). For a recent review discussing biotherapeutic proteins and their production, see Ghaderi et al., "Production platforms for biotherapeutic glycoproteins. Occurrence, impact, and challenges of non-human sialylation," 28 Biotechnol Genet Eng Rev. 147-75 (2012). In some embodiments, proteins contain modifications, adducts, and other covalently linked moieties. Those modifications, adducts and moieties include for example avidin, streptavidin, biotin, glycans (*e.g.*, N-acetylgalactosamine, galactose, neuraminic acid, N-acetylglucosamine, fucose, mannose, and other monosaccharides), PEG, polyhistidine, FLAG-tag, maltose binding protein (MBP), chitin binding protein (CBP), glutathione-S-transferase (GST) myc-epitope, fluorescent labels and other dyes, and the like. A biphasic pharmaceutical formulation of the present invention comprises at least 100 mg/ml of a VEGF antagonist.

Antibodies are often used as therapeutic biomolecules. The term "antibody", as used herein, includes immunoglobulin molecules comprised of four polypeptide chains, two heavy (H) chains and two light (L) chains inter-connected by disulfide bonds.

The phrase "Fc-containing protein" includes antibodies, bispecific antibodies, immunoadhesins, and other binding proteins that comprise at least a functional portion of an immunoglobulin CH2 and CH3 region. A "functional portion" refers to a CH2 and CH3 region that can bind a Fc receptor (e.g., an Fc$\gamma$R; or an FcRn, i.e., a neonatal Fc receptor), and/or that can participate in the activation of complement. If the CH2 and CH3 region contains deletions, substitutions, and/or insertions or other modifications that render it unable to bind any Fc receptor and also unable to activate complement, the CH2 and CH3 region is not functional.

Fc-containing proteins can comprise modifications in immunoglobulin domains, including where the modifications affect one or more effector function of the binding protein (e.g., modifications that affect Fc$\gamma$R binding, FcRn binding and thus half-life, and/or CDC activity).

VEGF antagonists include small molecules that inhibit VEGF-stimulated tyrosine kinases, including for example lapatinib, sunitinib, sorafenib, axitinib, and paxopanib (Yadav, 2015). Macromolecular inhibitors of VEGF include the monoclonal antibody bevacizumab, the Fab fragment ranibizumab, the trap aflibercept, the trap conbercept, and the PEGylated aptamer pegaptanib.

According to some embodiments, the second molecule (a.k.a. "molecule that reduces solubility" of the biphasic system) reduces the solubility of the first molecule (*i.e.*, the biotherapeutic or other drug or biomolecule). While "solubility" usually refers to an inherent physical property of a solute in a given solvent, dependent upon variables such as temperature, pressure, and pH, here "solubility" has a broader meaning. As used herein, "solubility" denotes the concentration of solute in a solvent. As such, solubility is affected by the addition or subtraction of other reagents, such as *inter alia* a salt, an additional solvent, and/or non-ionic macromolecules or other macromolecules that serve to exclude solvent and effectively reduce the amount of solvent available to dissolve the solute.

In an embodiment of the invention, a "molecule that reduces solubility" includes a "non-ionic macromolecule" (a.k.a. "water soluble non-ionic polymer"). These molecules act in part by excluding solvent and reducing the overall amount of water available for protein solvation. Non-ionic macromolecules include *e.g.*, polyethylene glycol (PEG), dextran, alginate, pectinate, carboxymethyl cellulose or starch, hydroxyethyl cellulose or starch, hydroxypropyl cellulose or starch, methyl cellulose or starch, polyacrylic and polymetaacrylic acids, polyethyleneimine, polyacrylamide, polyoxyethylene, polyvinylpyrrolidone (PVP), FICOLL®, PERCOLL®, and the like.

"Polyethylene glycol" or "PEG" refers to one example of a non-ionic macromolecule that is useful as a molecule that reduces the solubility of a biomolecule. PEG is a polyether polymer of ethylene oxide commonly used in food, medicine and cosmetics. PEG is commercially available in different molecular weights ranging from 300 g/mol to 10,000,000 g/mol. In an aspect of the invention, PEG 20000, PEG 8000 and PEG 3350 are useful. PEG having a molecular weight of 3350 grams/mole (PEG3350) is preferred for use in the present invention. PEG is available in different geometries, including linear, branched (3-10 chains attached to central core), star-shaped (10-100 chains attached to a central core), and comb-shaped (multiple chains attached to a polymer backbone).

"Dextran" is another non-ionic macromolecule exemplar that may be useful as a molecule that reduces the solubility of a biomolecule. Dextran is a branched chain glucan that is often used *inter alia* as a volume expander. It is generally recognized as safe and has been used in other applications to stabilize proteins.

In some embodiments, the first molecule, when in combination with the second molecule, exists in two solubility states, (1) in solution and (2) not in solution. The terms "dissolved", "solute", "soluble", "solubilized", "soluble fraction", "soluble part", "soluble phase", "saturated solution" each refer to portion of the totality of the first molecule that is in solution. The terms "solid", "precipitate", "non-soluble fraction", "non-soluble part", "insoluble fraction", "insoluble", "insoluble phase" each refer to the portion of the totality of the first molecule that is not in solution.

In one aspect of an open biphasic system, reducing the solubility of the drug with PEG enhances the overall long-term stability of the first molecule. The term "stability" refers to the retention of an acceptable degree of physical structure (colloidal, nature), chemical structure or biological function of the first molecule over time after deposition into a physiologically relevant environment such as the soluble fraction of a reservoir chamber. The first molecule may be stable even though it does not maintain 100% of its structure or function after storage or deposition for a defined amount of time. Under certain circumstances, if about 80%, about 85%, about 90%, about 95%, about 96%, about 97%, about 98% or about 99% of the first molecules have a native conformation, structure or function, the protein and formulation in the soluble fraction may be regarded as "stable".

Stability can be measured, *inter alia,* by determining the percentage of native molecule that remains in the formulation after storage or deposition for a defined amount of time at a defined temperature. The percentage of native molecule can be determined by, *inter alia,* size exclusion chromatography (*e.g.*, size exclusion high performance liquid chromatography [SE-HPLC]), such that native means non-aggregated and non-degraded. In certain embodiments, at least about 80%, 85%, 90%, 91%, 92%, 93%, 94%, 95%, 96%, 97%, 98%, 99% or 100% of the native form of the first molecule can be detected in the formulation after a defined amount of time at a defined temperature or under physiological conditions after deposition (e.g., implantation). The defined amount of time after which stability is measured can be about 14 days, about 28 days, about 1 month, about 2 months, about 3 months, about 4 months, about 5 months, about 6 months, about 7 months, about 8 months, about 9 months, about 10 months, about 11 months, about 12 months, about 18 months, about 24 months, or more. The temperature at which the device containing the first and second molecules may be kept when assessing stability can be any temperature from about -80°C to about 45°C, *e.g.,* storage at about -80°C, about -30°C, about -20°C, about 0°C, about 4°-8°C, about 5°C, about 25°C, about 35°C, about 37°C or other physiological temperatures, or about 45°C. For example, the first molecule may be deemed stable if after 3 months under physiological conditions, greater than about 75%, 80%, 85% or 90% of native molecule is detected in the soluble fraction by SE-HPLC or other size exclusion or size determination method. "Physiological temperature" includes the body temperature of any vertebrate. For example, the physiological temperature of humans is about 37°C. In some embodiments of the invention, physiological temperature is between about 25°C and about 45°C. In some embodiments, physiological temperature is about 25°C, about 26°C, about 27°C, about 28°C, about 29°C, about 30°C, about 31°C, about 32°C, about 33°C, about 34°C, about 35°C, about 36°C, about 37°C, about 38°C, about 39°C, about 40°C, about 41°C, about 42°C, about 43°C, about 44°C, and about 45°C.

Stability can be measured, *inter alia,* by determining the percentage of first molecule, such as a protein, that forms an aggregate (*i.e.*, high molecular weight species) within the reservoir device in the presence of second molecule, such as PEG, after a defined amount of time at a defined temperature, wherein stability is inversely proportional to the percent high molecular weight (HMW) species that is formed in the soluble fraction. Stability of the protein can be measured after its release from an open biphasic system where it was in the presence of a second molecule, such as PEG. The percentage of HMW species of the first molecule in the soluble fraction can be determined by, *inter alia,* size exclusion chromatography, as described above. A pharmaceutical formulation may also be deemed stable if after three months at physiological conditions less than about 15%, 14%, 13%, 12%, 11%, 10%, 9%, 8%, 7%, 6%, 5%, 4%, 3%, 2%, 1%, 0.5%, or 0.1% of the first molecule is detected in a HMW form.

Stability can be measured, *inter alia,* by determining the percentage of a first molecule, such as a protein, that is degraded or otherwise is found as a low molecular weight (LMW) species after release from an open biphasic system where it was in the presence of a second molecule, PEG, after a defined amount of time at a defined temperature. Stability is inversely proportional to the percent LMW species that is formed in the soluble fraction. The percentage of LMW species of the first molecule in the soluble fraction can be determined by, *inter alia,* size exclusion chromatography, as described above. A pharmaceutical formulation may also be deemed stable if after three months at physiological conditions less than about 15%, 14%, 13%, 12%, 11%, 10%, 9%, 8%, 7%, 6%, 5%, 4%, 3%, 2%, 1%, 0.5%, or 0.1% of the first molecule is detected in a LMW form.

In some embodiments, the open biphasic drug system is contained within a "reservoir device". The term "reservoir device" encompasses any container that contains the biphasic drug, partially sequesters the biphasic drug from the surrounding environment, and permits the efflux of soluble drug through a port at a pre-determined rate. The reservoir device represents an open and regulated system comprising a biphasic system. A closed biphasic system may be contained in another container that is not a reservoir device. For example, a biphasic system in a vial that is not in contact with another solution is a closed system, since the soluble molecule cannot diffuse away. The stability of the biphasic drug over time can be greater in an open system than in a closed system.

In some embodiments of the invention, the biphasic system contains an additional ingredient or excipient. "Excipients" include various substances used for various purposes including buffering, solubilizing, stabilizing, and/or protecting the drug, and maintaining or adjusting tonicity of the formulation. Protectants protect against thermal stress and/or physical stress like agitation. Buffers are well known in the art.

In some embodiments a buffer is included in the subject biphasic formulation. In some embodiments, the subject biphasic formulation contains a buffer at a concentration of about 1 mM to about 100 mM, about 5 mM to about 50 mM, or about 10 mM to about 20 mM. In some embodiments, the buffer is included in subject biphasic formulation at a concentration of 5 mM ± 0.75 mM to 15 mM ± 2.25 mM; 6 mM ± 0.9 mM to 14 mM ± 2.1 mM; 7 mM ± 1.05 mM to 13 mM ± 1.95 mM; 8 mM ± 1.2 mM to 12 mM ± 1.8 mM; 9 mM ± 1.35 mM to 11 mM ± 1.65 mM; 10 mM ± 1.5 mM; or about 10 mM. In some embodiments, the buffer is histidine, phosphate, carbonate, succinate, and/or acetate.

In some embodiments, the buffer is selected from a chemical capable of buffering somewhere within the pH range of about 3 to about 9, or within the pH range of about 3.7 to about 8.0. For example, the biphasic formulation may have a pH of about 3.4, about 3.6, about 3.8, about 4.0, about 4.2, about 4.4, about 4.6, about 4.8, about 5.0, about 5.2, about 5.4, about 5.6, about 5.8, about 6.0, about 6.2, about 6.4, about 6.6, about 6.8, about 7.0, about 7.2, about 7.4, about 7.6, about 7.8, or about 8.0.

The buffer may be a combination of individual buffers, such as, e.g., the combination of histidine and acetate (his-acetate buffer). In one embodiment, the buffer has a buffering range of about 3.5 to about 6, or about 3.7 to about 5.6, such as the range buffered by acetate. In one embodiment, the buffer has a buffering range of about 5.5 to about 8.5, or about 5.8 to about 8.0, such as the range buffered by phosphate. In one embodiment, the buffer has a buffering range of about 5.0 to about 8.0, or about 5.5 to about 7.4, such as the range buffered by histidine.

Excipients include stabilizers. As used herein, a stabilizer may be added to the subject biphasic formulation to stabilize the protein against aggregation or other degradation. Stabilizers that may be included in the subject biphasic formulation include polyols, sugars, salts (e.g., sodium chloride), amino acids, and the like. In some cases, the stabilizer can also act as a tonicifier that adjusts the osmolality of the biphasic formulation to match the osmolality of the physiological environment into which the biphasic formulation is deposited. For example, sodium chloride and sucrose each function as a tonicifier. Various individual stabilizers may be used alone or combined with one or more other stabilizers for optimal stabilizing or tonicifying effect. For example, a polyol may be combined with a sugar, a sugar with an amino acid, a polyol with an amino acid, a salt with a sugar, a salt with an amino acid, a salt with a polyol, and the like.

Polyols are organic molecules with more than one hydroxyl groups (-OH). Polyols include monomers as well as polymers. Sugar alcohols are a subgroup of polyols Sugar alcohols, which can serve as useful stabilizers, include mannitol, xylitol, sorbitol, isomalt, erythritol, maltitol, and glycerol. Other monomeric polyols include ethylene glycol, propylene glycol and pentaerythritol. Polymeric polyols may be polyesters or polyethers of polyol subunits. Useful exemplar polymeric polyols include polypropylene glycol, polyethylene glycol, and poly (tetramethylene ether) glycol.

Sugars like trehalose and sucrose may be used as stabilizers in the subject biphasic formulation. In some embodiments, sucrose is included in the subject biphasic formulation at a concentration of about 0.5% (w/v) to about 25% (w/v). In one embodiment, sucrose is included in the subject biphasic formulation at a concentration of about 0.5% (w/v), about 1% (w/v), about 1.5% (w/v), about 2% (w/v), about 2.5% (w/v), about 3% (w/v), about 3.5% (w/v), about 4% (w/v), about 4.5% (w/v), about 5% (w/v), about 6% (w/v), about 7% (w/v), about 8% (w/v), about 9% (w/v), about 10% (w/v), about 11% (w/v), about 12% (w/v), about 13% (w/v), about 14% (w/v), about 15% (w/v), about 16% (w/v), about 17% (w/v), about 18% (w/v), about 19% (w/v), about 20% (w/v), about 21% (w/v), about 22% (w/v), about 23% (w/v), about 24% (w/v), or about 25% (w/v).

Amino acids may also be included as stabilizers or tonicifying agents in the subject biphasic formulation. Useful amino acids include glycine, arginine, alanine, and proline. In some embodiments, arginine is used as a stabilizer and a viscosity reducer.

In some cases, one or more surfactants may be employed as an excipient in the subject biphasic formulation. Surfactants provide additional stability by reducing protein-protein hydrophobic interaction and the resulting formation of high molecular weight species (i.e., aggregates). Exemplary non-ionic surfactants that can be included in the subject biphasic formulation include, e.g., alkyl poly(ethylene oxide), alkyl polyglucosides (e.g., octyl glucoside and decyl maltoside), fatty alcohols such as cetyl alcohol and oleyl alcohol, cocamide MEA, cocamide DEA, and cocamide TEA. Specific non-ionic surfactants that can be included in the pre-lyophilized aqueous solution (or post reconstituted solution) include, e.g., polyoxyethylene sorbitan esters (a.k.a. polysorbates) such as polysorbate 20, polysorbate 28, polysorbate 40, polysorbate 60, polysorbate 65, polysorbate 80, polysorbate 81, and polysorbate 85; poloxamers such as poloxamer 188, poloxamer 407; polyethylene-polypropylene glycol; or polyethylene glycol (PEG). Polysorbate 20 is also known as TWEEN 20, sorbitan monolaurate and polyoxyethylenesorbitan monolaurate. Polysorbate 80 is also known as TWEEN 80, sorbitan monooleate and polyoxyethylenesorbitan monooleate.

In some embodiments, polysorbate 20 or polysorbate 80 may be included in the subject biphasic formulation at a concentration of about 0.001% (w/v) to about 0.5% (w/v). In some embodiments, the subject biphasic formulation contains about 0.001%; about 0.0015%; about 0.002%; about 0.0025%; about 0.003%; about 0.0035%; about 0.004%; about 0.0045%; about 0.005%; about 0.0055%; about 0.006%; about 0.0065%; about 0.007%; about 0.0075%; about 0.008%; about 0.0085%; about 0.009%; about 0.0095%; about 0.01%; about 0.015%; about 0.016%; about 0.017%; about 0.018%; about 0.019%; about 0.02%; about 0.021%; about 0.022%; about 0.023%; about 0.024%; about 0.025%; about 0.026%; about 0.027%; about 0.028%; about 0.029%; about 0.03%; about 0.031%; about 0.032%; about 0.033%; about 0.034%; about 0.035%; about 0.036%; about 0.037%; about 0.038%; about 0.039%; about 0.04%; about 0.041%; about 0.042%; about 0.043%; about 0.044%; about 0.045%; about 0.046%; about 0.047%; about 0.048%; about 0.049%; about 0.05%; about 0.051%; about 0.052%; about 0.053%; about 0.054%; about 0.055%; about 0.056%; about 0.057%; about 0.058%; about 0.059%; about 0.06%; about 0.061%; about 0.062%; about 0.063%; about 0.064%; about 0.065%; about 0.066%; about 0.067%; about 0.068%; about 0.069%; about 0.07%; about 0.071%; about 0.072%; about 0.073%; about 0.074%; about 0.075%; about 0.076%; about 0.077%; about 0.078%; about 0.079%; about 0.08%; about 0.081%; about 0.082%; about 0.083%; about 0.084%; about 0.085%; about 0.086%; about 0.087%; about 0.088%; about 0.089%; about 0.09%; about 0.091%; about 0.092%; about 0.093%; about 0.094%; about 0.095%; about 0.096%; about 0.097%; about 0.098%; about 0.099%; about 0.10%; about 0.15%; about 0.20%; about 0.25%; about 0.30%; about 0.35%; about 0.40%; about 0.45%; or about 0.50% polysorbate 20 or polysorbate 80.

**Embodiments**

In one aspect, the invention provides a stable biphasic pharmaceutical formulation containing a biomolecule and a molecule that limits the solubility of the biomolecule. The biomolecule is present as a biphasic system in the formulation where some of the biomolecule is in a soluble form (dissolved) and some is in an insoluble form (precipitate). In one embodiment, the formulation may be a closed system, where the soluble biomolecule does not have access to an outside aqueous environment, or in an open system where the soluble biomolecule has access to an environment with a lower concentration of the biomolecule. In some embodiments of the open system, the composition is contained within a reservoir chamber, which is housed in a reservoir device and contained by a porous structure. The reservoir device is open to the environment via a semi-permeable membrane or other porous structure, and may be implanted into a patient to deliver a therapeutically effective amount of the biomolecule to the therapeutic target in the patient.

In one aspect, the invention provides a biphasic pharmaceutical formulation containing at least 25 mg/mL of polyethylene glycol (PEG), at least 100 mg/mL of a protein, a soluble phase containing less than 50% of the total amount of protein and an amount of PEG, and an insoluble phase comprising insoluble protein. The soluble phase protein is stable for at least 30 days at physiological temperature, and has access through a semi-permeable membrane or other porous structure to an environment with a lower concentration of the protein (*i.e.*, an open system). In particular, the present invention provides A biphasic pharmaceutical formulation comprising:

a. at least 25 mg/mL of polyethylene glycol (PEG);

b. at least 100 mg/mL of a VEGF antagonist, wherein less than 50% of the VEGF antagonist is present in a dissolved form in a liquid phase with PEG, and the remainder of the VEGF antagonist is present in a solid precipitate form in equilibrium with the dissolved form, wherein the solid precipitate form is excluded from the liquid phase by PEG; and;

c. a semi-permeable membrane, wherein the semi-permeable membrane surrounds the liquid phase and preferentially retains PEG while allowing diffusion of the VEGF antagonist through the membrane, wherein said VEGF antagonist has at least 80% native conformation, structure or function in the liquid phase for at least 30 days at physiological temperature, and said liquid phase has access to an environment with a lower concentration of said VEGF antagonist through the semi-permeable membrane.

In some embodiments, the biphasic pharmaceutical formulation is for use in a method wherein it is administered to a patient or otherwise placed into an outside environment, where over time the soluble protein diffuses down a concentration gradient through the porous structure and into the environment. The total amount of the protein in the subject biphasic pharmaceutical formulation therefore declines over time and eventually becomes depleted. Therefore, in some embodiments the amount of protein in the formulation is less than the initial 100 mg/mL or more amount, depending on how long the formulation has accessed the environment with the lower protein concentration. In some embodiments, the total amount of protein in the subject biphasic pharmaceutical formulation will be between 0.1 mg/mL and 500 mg/mL of a protein over the lifetime a device of the present invention is used in a patient.

In some embodiments, the protein of the subject biphasic pharmaceutical formulation has a molecular mass of about 10 kD (i.e., 10,000 Daltons or 10,000 grams per mole) to about 200 kD, about 25 kD to about 160 kDa, about 10 kD to about 15 kD, about 15 kD to about 20 kDa, about 20 kD to about 25 kDa, about 25 kD to about 30 kDa, about 30 kD to about 35 kDa, about 35 kD to about 40 kDa, about 40 kD to about 45 kDa, about 45 kD to about 50 kDa, about 50 kD to about 55 kDa, about 55 kD to about 60 kDa, about 60 kD to about 65 kDa, about 65 kD to about 70 kDa, about 75 kD to about 80 kDa, about 80 kD to about 85 kDa, about 85 kD to about 90 kDa, about 90 kD to about 95 kDa, about 95 kD to about 100 kDa, about 100 kD to about 105 kDa, about 105 kD to about 110 kDa, about 110 kD to about 115 kDa, about 115 kD to about 120 kDa, about 120 kD to about 125 kDa, about 125 kD to about 130 kDa, about 130 kD to about 135 kDa, about 135 kD to about 140 kDa, about 140 kD to about 145 kDa, about 145 kD to about 150 kDa, about 150 kD to about 155 kDa, about 155 kD to about 160 kDa, about 160 kD to about 165 kDa, about 165 kD to about 170 kDa, about 170 kD to about 175 kDa, about 175 kD to about 180 kDa, about 180 kD to about 185 kDa, about 185 kD to about 190 kDa, about 190 kD to about 195 kD, or about 195 kD to about 200 kD. In some embodiments, the protein is a nanobody or a similar-sized protein having a molecular weight of about 12-15 kD. In some embodiments, the protein is an antibody fragment such as a Fab or a similar-sized protein having a molecular weight of about 50 kD. In some embodiments, the protein is a single-chain variable fragment (scFv) or a similar-sized protein having a molecular weight of about 25 kD. In some embodiments, the protein is a receptor-Fc-fusion protein or a similar-sized protein having a molecular weight between about 90 kD and 110 kD. In some embodiments, the protein is an antibody or a similar-sized protein having a molecular weight of between about 150 kD and about 160 kD.

The protein is a VEGF antagonist, such as aflibercept, which is a trap molecule, ranibizumab and bevacizumab, which are antibodies Also referred to as a biomolecule is a PDGF antagonist, which may be e.g., a small molecule inhibitor, a soluble receptor, a trap molecule or other fusion protein, an anti-PDGF antibody, an RNA, or an aptamer. Also referred to as a biomolecule is an Ang2 antagonist, which may be e.g., a small molecule inhibitor, a soluble receptor, a trap molecule or other fusion protein, an anti-Ang2 antibody, an RNA, or an aptamer.

In some embodiments, the total concentration of the protein in the subject biphasic pharmaceutical formulation is between about 100 mg/mL and 1,400 mg/mL or more. In some embodiments, dry protein (*e.g.*, lyophilized or spray-dried) is combined with PEG. The total amount of protein that can fit within a given volume depends upon the density of the solid protein and the amount of PEG and other excipients included with the protein in the mixture. US Patent Application No. 2013/0324942 shows doses and concentrations of drugs in a device. The volume available in the device will limit to some extent the protein concentration that can be put into a device. The upper bound will be defined by the density of the protein powder and limitations about much can be packed into the reservoir volume (which will depend on the protein formulation). US Patent No. 8,623,395 describes device volumes and protein content, such as VEGF antagonists, including aflibercept.

In some embodiments, the total concentration of the protein may exceed 1,400 mg/mL. In some embodiments, the total protein or other biomolecule concentration is about 100 mg/mL, 110 mg/mL, about 120 mg/mL, about 130 mg/mL, about 140 mg/mL, about 150 mg/mL, about 160 mg/mL, about 170 mg/mL, about 180 mg/mL, about 190 mg/mL, about 200 mg/mL, about 210 mg/mL, about 220 mg/mL, about 230 mg/mL, about 240 mg/mL, about 250 mg/mL, about 260 mg/mL, about 270 mg/mL, about 280 mg/mL, about 290 mg/mL, about 300 mg/mL, about 310 mg/mL, about 320 mg/mL, about 330 mg/mL, about 340 mg/mL, about 350 mg/mL, about 360 mg/mL, about 370 mg/mL, about 380 mg/mL, about 390 mg/mL, about 400 mg/mL, about 410 mg/mL, about 420 mg/mL, about 430 mg/mL, about 440 mg/mL, about 450 mg/mL, about 460 mg/mL, about 470 mg/mL, about 480 mg/mL, about 490 mg/mL, , about 500 mg/mL, about 510 mg/mL, about 520 mg/mL, about 530 mg/mL, about 540 mg/mL, about 550 mg/mL, about 560 mg/mL, about 570 mg/mL, about 580 mg/mL, about 590 mg/mL, about 600 mg/mL, about 610 mg/mL, about 620 mg/mL, about 630 mg/mL, about 640 mg/mL, about 650 mg/mL, about 660 mg/mL, about 670 mg/mL, about 680 mg/mL, about 690 mg/mL, about 700 mg/mL, about 710 mg/mL, about 720 mg/mL, about 730 mg/mL, about 740 mg/mL, about 750 mg/mL, about 760 mg/mL, about 770 mg/mL, about 780 mg/mL, about 790 mg/mL, about 800 mg/mL, about 810 mg/mL, about 820 mg/mL, about 830 mg/mL, about 840 mg/mL, about 850 mg/mL, about 860 mg/mL, about 870 mg/mL, about 880 mg/mL, about 890 mg/mL, about 900 mg/mL, about 910 mg/mL, about 920 mg/mL, about 930 mg/mL, about 940 mg/mL, about 950 mg/mL, about 960 mg/mL, about 970 mg/mL, about 980 mg/mL, about 990 mg/mL, about 1000 mg/mL, about 1025 mg/mL, about 1050 mg/mL, about 1075 mg/mL, about 1100 mg/mL, about 1125 mg/mL, about 1150 mg/mL, about 1175 mg/mL, about 1200 mg/mL, about 1225 mg/mL, about 1250 mg/mL, about 1275 mg/mL, about 1300 mg/mL, about 1325 mg/mL, about 1350 mg/mL, about 1375 mg/mL, about 1400 mg/mL, or about 1425 mg/mL. In some embodiments, the total protein concentration is greater than 1400 mg/mL.

In some embodiments, the concentration of protein in the soluble phase of the subject biphasic pharmaceutical formulation is about 25 mg/mL to about 100 mg/mL. In some embodiments, the concentration of the protein or other biomolecule in the soluble phase is about 25 mg/mL, about 26 mg/mL, about 27 mg/mL, about 28 mg/mL, about 29 mg/mL, about 30 mg/mL, about 31 mg/mL, about 32 mg/mL, about 33 mg/mL, about 34 mg/mL, about 35 mg/mL, about 36 mg/mL, about 37 mg/mL, about 38 mg/mL, about 39 mg/mL, about 40 mg/mL, about 41 mg/mL, about 42 mg/mL, about 43 mg/mL, about 44 mg/mL, about 45 mg/mL, about 46 mg/mL, about 47 mg/mL, about 48 mg/mL, about 49 mg/mL, about 50 mg/mL, about 51 mg/mL, about 52 mg/mL, about 53 mg/mL, about 54 mg/mL, about 55 mg/mL, about 56 mg/mL, about 57 mg/mL, about 58 mg/mL, about 59 mg/mL, about 60 mg/mL, about 61 mg/mL, about 62 mg/mL, about 63 mg/mL, about 64 mg/mL, about 65 mg/mL, about 66 mg/mL, about 67 mg/mL, about 68 mg/mL, about 69 mg/mL, about 70 mg/mL, about 71 mg/mL, about 72 mg/mL, about 73 mg/mL, about 74 mg/mL, about 75 mg/mL, about 76 mg/mL, about 77 mg/mL, about 78 mg/mL, about 79 mg/mL, about 80 mg/mL, about 81 mg/mL, about 82 mg/mL, about 83 mg/mL, about 84 mg/mL, about 85 mg/mL, about 86 mg/mL, about 87 mg/mL, about 88 mg/mL, about 89 mg/mL, about 90 mg/mL, about 91 mg/mL, about 92 mg/mL, about 93 mg/mL, about 94 mg/mL, about 95 mg/mL, about 96 mg/mL, about 97 mg/mL, about 98 mg/mL, about 99 mg/mL, or about 100 mg/mL. In some embodiments, the protein or other biomolecule in the soluble phase may be greater than 100 mg/mL.

In the biphasic formulation, the proportion of the protein in the soluble phase relative to the total amount of protein in the biphasic formulation (both soluble and insoluble phases) is less than 50%. In some embodiments, the proportion of the protein in the soluble phase relative to the total amount of protein in the biphasic formulation is about 50%, about 49%, about 48%, about 47%, about 46%, about 45%, about 44%, about 43%, about 42%, about 41%, about 40%, about 39%, about 38%, about 37%, about 36%, about 35%, about 34%, about 33%, about 32%, about 31%, about 30%, about 29%, about 28%, about 27%, about 26%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1%, between about 0.1% and about 1%, or less than 0.1%.

In some embodiments, the PEG is present in the composition at a concentration of about 25 mg/mL to about 150 mg/mL In some embodiments, the concentration of PEG in the composition is about 25 mg/mL, about 30 mg/mL, about 35 mg/mL, about 40 mg/mL, about 45 mg/mL, about 50 mg/mL, about 60 mg/mL, about 65 mg/mL, about 70 mg/mL, about 75 mg/mL, about 80 mg/mL, about 85 mg/mL, about 90 mg/mL, about 95 mg/mL, about 100 mg/mL, about 105 mg/mL, about 110 mg/mL, about 115 mg/mL, about 120 mg/mL, about 125 mg/mL, about 130 mg/mL, about 135 mg/mL, about 140 mg/mL, about 145 mg/mL, or about 150 mg/mL.

In some embodiments, sucrose is included in the subject biphasic formulation at a concentration of about 0.5% (w/v) to about 25% (w/v). In one embodiment, sucrose is included in the subject biphasic formulation at a concentration of about 0.5% (w/v), about 1% (w/v), about 1.5% (w/v), about 2% (w/v), about 2.5% (w/v), about 3% (w/v), about 3.5% (w/v), about 4% (w/v), about 4.5% (w/v), about 5% (w/v), about 6% (w/v), about 7% (w/v), about 8% (w/v), about 9% (w/v), about 10% (w/v), about 11% (w/v), about 12% (w/v), about 13% (w/v), about 14% (w/v), about 15% (w/v), about 16% (w/v), about 17% (w/v), about 18% (w/v), about 19% (w/v), about 20% (w/v), about 21% (w/v), about 22% (w/v), about 23% (w/v), about 24% (w/v), or about 25% (w/v).

In another aspect, the invention provides a drug delivery device for the extended delivery of a drug which remains stable throughout an extended period of time at physiological temperature. The subject drug delivery device contains the subject stable biphasic pharmaceutical formulation, a reservoir chamber that contains the stable biphasic pharmaceutical formulation, a housing that encompasses the reservoir chamber, and a semi-permeable membrane or other porous structure in the housing that permits access of the soluble phase protein to an environment with a lower concentration of the protein.

In one embodiment, the subject drug delivery device has a volume of about 5 μl to about 50 μl, about 10 μl to about 25 μl, about 5 μl, about 6 μl, about 7 μl, about 8 μl, about 9 μl, about 10 μl, about 11 μl, about 12 μl, about 13 μl, about 14 μl, about 15 μl, about 16 μl, about 17 μl, about 18 μl, about 19 μl, about 20 μl, about 21 μl, about 22 μl, about 23 μl, about

24 µl, about 25 µl, about 26 µl, about 27 µl, about 28 µl, about 29 µl, about 30 µl, about 31 µl, about 32 µl, about 33 µl, about 34 µl, about 35 µl, about 36 µl, about 37 µl, about 38 µl, about 39 µl, about 40 µl, about 41 µl, about 42 µl, about 43 µl, about 44 µl, about 45 µl, about 46 µl, about 47 µl, about 48 µl, about 49 µl, or about 50 µl.

In one embodiment, the subject reservoir chamber contains between about 0.1 mg and about 10 mg of the subject protein. In some embodiments, the reservoir chamber contains a total amount of protein at about 0.1 mg, about 0.2 mg, about 0.3 mg, about 0.4 mg, about 0.5 mg, about 0.6 mg, about 0.7 mg, about 0.8 mg, about 0.9 mg, about 1 mg, about 2 mg, about 3 mg, about 4 mg, about 5 mg, about 6 mg, about 7 mg, about 8 mg, about 9 mg, or about 10 mg.

In some embodiments, the subject reservoir chamber is of a size that permits insertion of the device into the vitreous of the eye, or other accommodating tissue.

In some embodiments, the subject reservoir chamber is of a size that permits subcutaneous placement of the device in a patient.

In one embodiment, the subject drug delivery device has a volume of about 25 µl to about 150 µl, about 25 µl, about 30 µl, about 35 µl, about 40 µl, about 45 µl, about 50 µl, about 55 µl, about 60 µl, about 65 µl, about 70 µl, about 75 µl, about 80 µl, about 85 µl, about 90 µl, about 95 µl, about 100 µl, about 105 µl, about 110 µl, about 115 µl, about 120 µl, about 125 µl, about 130 µl, about 135 µl, about 140 µl, about 145 µl, or about 150 µl.

In one embodiment, the subject reservoir chamber contains between about 25 mg and about 180 mg of the subject protein. In some embodiments, the reservoir chamber contains a total amount of protein at about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, about 100 mg, about 105 mg, about 110 mg, about 115 mg, about 120 mg, about 125 mg, about 130 mg, about 135 mg, about 140 mg, about 145 mg, about 150 mg, about 155 mg, about 160 mg, about 165 mg, about 170 mg, about 175 mg, or about 180 mg.

**Examples**

The present invention is illustrated by the following examples.

Example 1: Release and Stability of Protein in Protein and PEG (Biphasic) Mixture

Aflibercept formulations were developed containing 100 mg/ml to 300 mg/ml of protein that remained stable at 37°C for > 3 months. Aflibercept protein of varying total concentration in aqueous solution was combined with polyethylene glycol having a molecular weight of 3,350 grams per mole (PEG3350) of varying concentration in solution. In one experiment, 5 mg/ml to 50 mg/mL aflibercept was combined with 45 mg/ml to 150 mg/ml of PEG3350. The solubility of the liquid aflibercept combinations (closed circles) were plotted against PEG3350 concentration (Figure 10). The log solubility of liquid aflibercept was plotted against PEG3350 concentration (closed diamonds, Figure 11). All concentrations of aflibercept generated essentially the same solubility curve as represented in Figure 10.

In another experiment, freeze-dried aflibercept was combined with dry PEG3350 (with sucrose included), and then reconstituted to attain the experimental PEG3350 concentration. The results are depicted in Figure 10 (open circles) and Figure 11 (closed squares), showing the reduction in aflibercept solubility with increasing PEG concentration. The difference in slope (*i.e.,* solubility) between the reconstituted solid protein (closed squares at Figure 11) and the initial liquid protein (closed diamonds at Figure 11) is likely due to the difference in sucrose content and not due to the physical state of the starting protein as solid or liquid. The solid aflibercept included sucrose, whereas the liquid aflibercept did not. At 30 mg/ml PEG3350, the solubility of aflibercept is not affected (*e.g.*, it is at least 45 mg/ml). The concentration of soluble aflibercept was determined as well as the portion of total aflibercept that retains its native conformation. Aflibercept purity remained greater than 95% in all solutions (liquid and solid reconstituted) with PEG3350 between 30 and 150 mg/ml, as determined by ultrahigh performance size exclusion chromatography (UP-SEC).

To test whether the aflibercept that was precipitated (liquid formulation experiment) or excluded from solution (reconstituted experiment) could be subsequently solubilized and retain native conformation, the aflibercept/PEG mixture was diluted from 150 mg/ml PEG, to 30 mg/ml PEG, which was previously determined to not limit aflibercept solubility. Here, essentially all of the aflibercept was recovered, with more than 98% having native conformation (Table 2).

TABLE 2

| Summary | % HMW | % Native | %LMW | % Recovery |
|---|---|---|---|---|
| Liquid | 1.14 | 98.86 | 0 | 103 |
| Reconstituted | 1.34 | 98.66 | 0 | 109 |

Example 2: Controlled Release Reservoir Device

A reservoir device for the controlled release of a protein was manufactured from permeable thin-film polycaprolactone (PCL). The housing of the device contained non-porous PCL, which is impermeable an antibody or a receptor-Fc-fusion dimer, and a strip of porous PCL comprising either nano-channels or micropores. The porous membranes were fabricated either by templating to form channels, or by using PEG as a porogen to form tortuous pores.

Methods of manufacturing pores in membranes are described in Bernards et al., "Nanostructured thin film polymer devices for constant-rate protein delivery," 12 NanoLett 5355-61 (2012); He et al., "Use of a nanoporous biodegradable miniature device to regulate cytokine release for cancer treatment," 151(3) J. Control Release 239-45 (2011); Gin and Noble, "Designing the Next Generation of Chemical Separation Membranes," 332 Science 674-676 (2011); Wirtz et al., "Transport properties of template synthesized gold and carbon nanotube membranes," 1(3,4) Int. J. Nanoscience 255 (2002); and Li et al., "Preparation and characterization of chitosan nanopores membranes for the transport of drugs," 420(2) Int. J. Pharm. 371-7 (2011).

The nanochannel porous PCL membrane ("nanoPCL") comprised approximately 20 nm diameter pores and was cast at a thickness of about 500 nm. The nanoPCL membrane was applied to a microporous backing layer for structural integrity. The microporous membrane ("mpPCL") contained pores having a cross-section diameter of about 1-2 microns with an interconnecting pore size estimated at < 1 micron. See Bernards, 2012.

An mpPCL thin film reservoir device was fabricated by casting both a non-porous PCL film and a porous PCL film. A strip of porous film was then spliced between two sections of porous film by heat sealing the joints. The resultant composite film (*i.e.*, a non-porous film containing a porous strip) was rolled into a cylinder (tube) using a rod-shaped template and heat sealed at one end, leaving the other end open. The drug substance (*infra*) was loaded into the open end, and then the open end was heat sealed. The PCL membrane was heat sealed using an electrically heated NiChrome wire, controlled by a power source, embedded within or between polydimethylsiloxane.

In one device, 3-4 mg of aflibercept protein was loaded into the PCL tube having a diameter of about 1-1.5 mm, and a length of about 8-10 mm.

Parameters affecting the rate of release of protein from the device were tested. Those parameters included membrane design (*i.e.*, nanoPCL vs. mpPCL), membrane thickness (28 $\mu$m, 63 $\mu$m, and 106 $\mu$m), and porous surface area (23 mm$^2$ *vs.* 60 mm$^2$). Release rates over the course of 50 days were approximately an order of magnitude faster for the mpPCL design compared to the nanoPCL design. Also, the surface area of the porous "window" significantly affected the release rate. A device having a 60 mm$^2$ area porous structure and loaded with 3 mg of aflibercept released about 105 $\mu$g of aflibercept per day. A device having a 23 mm$^2$ area porous structure and loaded with 3 mg of aflibercept protein released about 42 $\mu$g of aflibercept per day.

While the surface area of the window affected the release rate, membrane thickness did not. Thus, by altering the surface area of the porous structure, the release rate of protein can be controlled independent of device size. In this example, limiting the solubility of aflibercept to 10 mg/mL kept the reservoir concentration constant, allowing the protein release rate to be controlled by manipulating the porous area.

Example 3: Release of Protein from Reservoir Device

The rate of release of protein from a PCL reservoir device depended upon the concentration of soluble protein and the surface area of the porous structure. PCL devices loaded with the PEG3350 / aflibercept biphasic combination were exposed to an aqueous environment. The aflibercept released from the device was measured over time.

In one experiment, the concentration of aflibercept in the soluble phase varied from about 5 mg/ml to about 45 mg/ml depending upon the concentration of PEG3350. The surface area of the porous structure was kept constant at about 23 mm$^2$. Here, when the soluble phase concentration of aflibercept was about 6 mg/ml, the release rate was about 0.32 $\mu$g/day-mm$^2$. When the soluble phase concentration of aflibercept was about 46 mg/ml, the release rate was about 3.3 $\mu$g/day-mm$^2$ (Figure 12).

In another experiment, the concentration of the soluble phase aflibercept was kept constant at about 7 mg/ml with the concentration of PEG3350 at 70 mg/ml; and the surface area of the porous structure was varied from about 13 mm$^2$ to about 45 mm$^2$. Here, when the surface area of the porous window was about 13 mm$^2$, the protein was released at a rate of about 0.49 $\mu$g/day per mg/ml. When the surface area of the porous window was about 45 mm$^2$, the protein was released at a rate of about 4.9 $\mu$g/day per mg/ml (Figure 13).

The stability and release rates of soluble aflibercept from the biphasic aflibercept mixture in the PCL device were assessed over a period of time at 37°C in buffered physiologically isotonic solution. The PCL devices had a porous structure area of about 30 to 45 mm$^2$. Each device was loaded with about 2.7 mg to 3.7 mg of aflibercept. In one experiment, the concentration of PEG3350 was about 100 mg/ml and the concentration of the soluble phase aflibercept was about 2 mg/ml (Figure 14A).

In another experiment, the concentration of PEG3350 was about 70 mg/ml and the concentration of the soluble phase aflibercept was about 7 mg/ml (Figure 14B). No significant difference between the release rates for the 70 mg/ml PEG and the 100 mg/ml PEG reservoirs was observed after 35 days under the model physiological conditions. The average release rate was about 40 μg/day ± 10 μg/day across both sets of experiments (Figures 15A and 15B).

In another experiment, the concentration of PEG3350 was 80 mg/mL ± 20 mg/mL and the total concentration (soluble plus insoluble) of aflibercept was between about 60 mg/mL and about 210 mg/mL. The rate of release of soluble aflibercept from the device ranged from about 0.03 mg to about 0.055 mg per day (Table 3). The stability of the soluble aflibercept released from the device over time was assessed by quantifying the relative amount of aflibercept monomer remaining in each time-point sample. The relative amount of monomer aflibercept is presented as percent (%) purity in Figure 15. Monomer was quantified by high performance size-exclusion chromatography. The percent purity of aflibercept released from the device containing PEG3350 (80 mg/mL ± 20 mg/mL) was compared to the percent purity of aflibercept released from a device containing only sucrose and no PEG. Approximately 85% of the aflibercept released from the device at 61 days remained monomeric. The results are depicted in Figure 15.

TABLE 3

| PEG Load (mg) | Drug Load (mg) | Device Area (mm$^2$) | Hydration Vol (ml) | Porous Area (mm$^2$) | mg/ml PEG | mg/ml Drug | mg/day release of Drug |
|---|---|---|---|---|---|---|---|
| 1.2 | 3.7 | 66 | 0.018 | 33 | 67 | 206 | 0.032 |
| 2.9 | 3.2 | 90 | 0.042 | 45 | 69 | 76 | 0.045 |
| 2.9 | 2.8 | 80 | 0.046 | 40 | 63 | 61 | 0.031 |
| 4 | 3.0 | 75 | 0.040 | 38 | 99 | 74 | 0.055 |
| 3.3 | 2.9 | 82 | 0.032 | 41 | 104 | 91 | 0.038 |

Example 4: Biphasic Formulation-Containing Nano/Microporous Device

A piece of microporous or nanoporous PCL film was spliced between two pieces of nonporous PCL film using heat sealing. Heat sealing was accomplished by aligning the film above a nickel-chromium wire embedded between two slabs of polydimethylsiloxane (PDMS). The wire was heated by passing a current through the wire. The film was then rolled around a cylindrical mold whose diameter was chosen based on the target device dimensions. The film was then heat sealed along the longitudinal axis of the cylinder and on one end on the cylinder to create a hollow cylinder with one open end. Lyophilized protein was compressed into a cylindrical pellet and loaded into the reservoir through the open end. PEG3350 was likewise pelleted and loaded through the open end of the cylinder.

Before and after each loading step, the device was weighed to determine the mass loaded. The device was then sealed along the open end using the same heat sealing method. The PEG3350 loading was calculated based on mass loaded and hydration volume, which was determined by calculating the difference in device weight before and after hydration.

Loaded devices were incubated in balanced salt solution-like (BSS-like) at pH 7.2 at 37°C. At each time point, the device was removed from the BSS-like media and then placed into a fresh aliquot of BSS-like media and returned to incubation. The release media from which the device was removed was retained for analysis. At each time point, samples were analyzed by UP-SEC to quantify the amount of protein release and the % purity of the protein. The % purity of the released protein is depicted in Figure 15. The rate of protein release correlated directly to soluble protein concentration (Figure 12).

With increasing PEG concentration, the solubility of the protein decreased and the stability of the protein increased to a minimum soluble concentration of about 0.05 mg/mL. By limiting the solubility of the protein in the device reservoir, the PEG limited the driving force of diffusion, i.e., lowering the soluble phase protein concentration in reservoir decreased the protein concentration differential between the physiological environment and the formulation, and reduced the rate of protein release from device reservoir. By limiting solubility, the PEG also maintained a constant protein concentration in the device reservoir until the total amount of remaining protein was less than the limit of solubility for the protein.

Example 5: In vivo release of Receptor-Fc-fusion Protein

Microporous PCL devices loaded with PEG and approximately 1.6 milligrams of biphasic aflibercept were implanted intravitreally in African green monkeys (Group 2). Control monkeys received microporous PCL devices not containing aflibercept (Group 1). Tissue samples were collected from the control group (Group 1) and the experimental group (Group 2) at different days post-implantation. The amount of aflibercept in each sample was determined using an ELISA with VEGF as the capture molecule.

There was no free or bound aflibercept detected in plasma samples collected from animals that received the drug-free device (Group 1) or in baseline plasma samples from animals that subsequently received a device loaded with aflibercept (Group 2). Free aflibercept was detected in at least one plasma sample collected from a Group 2 animal. Bound aflibercept was detected in the plasma samples collected from three of four Group 2 animals showing that active protein was released from the device and able to bind to the endogenous VEGF in the target tissue.

Free aflibercept was detected in aqueous humor samples collected from all four animals in Group 2. In the vitreous, bound aflibercepts was detected in at least one of the Group 2 animals and free aflibercept was detected in the choroid and retinal samples of at least one Group 2 animal. Free aflibercept was not detected in aqueous humor, choroid, retinal or vitreal samples collected from Group 1 animals.

These results show that aflibercept is released *in vivo* in the eye and moves into the target environment from a microporous device charged with biphasic aflibercept.

## Claims

1. A biphasic pharmaceutical formulation comprising:

   a. at least 25 mg/mL of polyethylene glycol (PEG);
   b. at least 100 mg/mL of a VEGF antagonist, wherein less than 50% of the VEGF antagonist is present in a dissolved form in a liquid phase with PEG, and the remainder of the VEGF antagonist is present in a solid precipitate form in equilibrium with the dissolved form, wherein the solid precipitate form is excluded from the liquid phase by PEG; and;
   c. a semi-permeable membrane, wherein the semi-permeable membrane surrounds the liquid phase and preferentially retains PEG while allowing diffusion of the VEGF antagonist through the membrane, wherein said VEGF antagonist has at least 80% native conformation, structure or function in the liquid phase for at least 30 days at physiological temperature, and said liquid phase has access to an environment with a lower concentration of said VEGF antagonist through the semi-permeable membrane.

2. The biphasic pharmaceutical formulation of claim 1, wherein said VEGF antagonist is between 100 mg/mL and 1400 mg/mL.

3. The biphasic pharmaceutical formulation of claim 1 or 2, wherein the concentration of PEG is between 25 mg/mL and 150 mg/mL.

4. The biphasic pharmaceutical formulation of claim 1 or 2, wherein the concentration of the VEGF antagonist is between 100 mg/mL and 500 mg/mL.

5. The biphasic pharmaceutical formulation of claim 1 or 2, wherein said liquid phase comprises between about 0.05 mg/mL and about 50 mg/mL of said VEGF antagonist in dissolved form.

6. The biphasic pharmaceutical formulation of claim 1 or 2, wherein said VEGF antagonist has a molecular weight of about 25 kD to about 160 kD.

7. The biphasic pharmaceutical formulation of claim 1 or 2, wherein said VEGF antagonist in the liquid phase is stable for at least 60 days at physiological temperature, wherein stability of the VEGF antagonist in the liquid phase is assessed by determining the percentage of native molecules using size exclusion high performance liquid chromatography (SE-HPLC).

8. The biphasic pharmaceutical formulation of claim 1 or 2, wherein at least about 85% of said VEGF antagonist in the liquid phase has a native molecular weight at 60 days at physiological temperature as determined by size-exclusion chromatography.

9. The biphasic pharmaceutical formulation of claim 1 or 2, wherein said VEGF antagonist in the liquid phase is more stable than an equivalent concentration of the liquid phase without PEG or in a closed biphasic system.

10. The biphasic pharmaceutical formulation of claim 1 or 2, wherein said environment is a physiological environment.

11. The biphasic pharmaceutical formulation of claim 1 or 2, wherein said biphasic pharmaceutical composition is contained in a reservoir device with a porous structure.

12. The formulation of claim 1, wherein said PEG is fully soluble, is only in the liquid phase, and is at a lower concentration in said liquid phase than said protein in said solid phase.

**Patentansprüche**

1. Zweiphasige pharmazeutische Formulierung, umfassend:

   a. wenigstens 25 mg/ml Polyethylenglykol (PEG);
   b. wenigstens 100 mg/ml eines VEGF-Antagonisten, wobei weniger als 50 % des VEGF-Antagonisten in einer gelösten Form in einer flüssigen Phase mit PEG vorliegt und der Rest des VEGF-Antagonisten in einer festen Niederschlagsform im Gleichgewicht mit der gelösten Form vorliegt, wobei die feste Niederschlagsform durch PEG aus der flüssigen Phase ausgeschlossen wird; und
   c. eine semipermeable Membran, wobei die semipermeable Membran die flüssige Phase umgibt und vorzugsweise PEG zurückhält, während sie die Diffusion des VEGF-Antagonisten durch die Membran ermöglicht, wobei der VEGF-Antagonist wenigstens 80 % native Konformation, Struktur oder Funktion in der flüssigen Phase für wenigstens 30 Tage bei physiologischer Temperatur aufweist, und die flüssige Phase Zugang zu einer Umgebung mit einer geringeren Konzentration des VEGF-Antagonisten durch die semipermeable Membran aufweist.

2. Zweiphasige pharmazeutische Formulierung nach Anspruch 1, wobei der VEGF-Antagonist zwischen 100 mg/ml und 1400 mg/ml liegt.

3. Zweiphasige pharmazeutische Formulierung nach Anspruch 1 oder 2, wobei die Konzentration von PEG zwischen 25 mg/ml und 150 mg/ml liegt.

4. Zweiphasige pharmazeutische Formulierung nach Anspruch 1 oder 2, wobei die Konzentration des VEGF-Antagonisten zwischen 100 mg/ml und 500 mg/ml liegt.

5. Zweiphasige pharmazeutische Formulierung nach Anspruch 1 oder 2, wobei die flüssige Phase zwischen etwa 0,05 mg/ml und etwa 50 mg/ml des VEGF-Antagonisten in gelöster Form umfasst.

6. Zweiphasige pharmazeutische Formulierung nach Anspruch 1 oder 2, wobei der VEGF-Antagonist ein Molekulargewicht von etwa 25 kD bis etwa 160 kD aufweist.

7. Zweiphasige pharmazeutische Formulierung nach Anspruch 1 oder 2, wobei der VEGF-Antagonist in der flüssigen Phase bei physiologischer Temperatur wenigstens 60 Tage lang stabil ist, wobei die Stabilität des VEGF-Antagonisten in der flüssigen Phase durch Bestimmen des Prozentsatzes an nativen Molekülen unter Verwendung von Größenausschluss-Hochleistungsflüssigkeitschromatographie (SE-HPLC) bewertet wird.

8. Zweiphasige pharmazeutische Formulierung nach Anspruch 1 oder 2, wobei wenigstens etwa 85 % des VEGF-Antagonisten in der flüssigen Phase nach 60 Tagen bei physiologischer Temperatur ein natives Molekulargewicht aufweist, wie durch Größenausschlusschromatographie bestimmt.

9. Zweiphasige pharmazeutische Formulierung nach Anspruch 1 oder 2, wobei der VEGF-Antagonist in der flüssigen Phase stabiler ist als eine äquivalente Konzentration der flüssigen Phase ohne PEG oder in einem geschlossenen zweiphasigen System.

10. Zweiphasige pharmazeutische Formulierung nach Anspruch 1 oder 2, wobei die Umgebung eine physiologische Umgebung ist.

11. Zweiphasige pharmazeutische Formulierung nach Anspruch 1 oder 2, wobei die zweiphasige pharmazeutische Zusammensetzung in einer Reservoirvorrichtung mit einer porösen Struktur enthalten ist.

12. Formulierung nach Anspruch 1, wobei das PEG vollständig löslich ist, sich nur in der flüssigen Phase befindet und in der flüssigen Phase in einer geringeren Konzentration vorliegt als das Protein in der festen Phase.

**Revendications**

1. Formulation pharmaceutique biphasique comprenant :

a. au moins 25 mg/mL de polyéthylène glycol (PEG),
b. au moins 100 mg/mL d'un antagoniste du VEGF, au moins 50 % de l'antagoniste du VEGF étant présent sous forme dissoute dans une phase liquide avec le PEG, et le restant de l'antagoniste du VEGF étant présent sous forme de précipité solide à l'équilibre avec la forme dissoute, la forme de précipité solide étant exclue de la phase liquide par le PEG, et
c. une membrane semi-perméable, la membrane semi-perméable enveloppant la phase liquide et retenant de préférence le PEG tout en permettant la diffusion de l'antagoniste du VEGF à travers celle-ci, ledit antagoniste du VEGF ayant au moins 80 % de sa conformation, structure ou fonction native dans la phase liquide pendant au moins 30 jours à température physiologique, et ladite phase liquide pouvant accéder à un environnement présentant une moindre concentration en ledit antagoniste du VEGF à travers la membrane semi-perméable.

2. Formulation pharmaceutique biphasique selon la revendication 1, dans laquelle ledit antagoniste du VEGF se situe entre 100 mg/mL et 1400 mg/mL.

3. Formulation pharmaceutique biphasique selon la revendication 1 ou 2, dans laquelle la concentration en PEG se situe entre 25 mg/mL et 150 mg/mL.

4. Formulation pharmaceutique biphasique selon la revendication 1 ou 2, dans laquelle la concentration en antagoniste du VEGF se situe entre 100 mg/mL et 500 mg/mL.

5. Formulation pharmaceutique biphasique selon la revendication 1 ou 2, dans laquelle ladite phase liquide comprend entre environ 0,05 mg/mL et environ 50 mg/mL dudit antagoniste du VEGF sous forme dissoute.

6. Formulation pharmaceutique biphasique selon la revendication 1 ou 2, dans laquelle ledit antagoniste du VEGF a un poids moléculaire d'environ 25 kD à environ 160 kD.

7. Formulation pharmaceutique biphasique selon la revendication 1 ou 2, dans laquelle ledit antagoniste du VEGF dans la phase liquide est stable pendant au moins 60 jours à température physiologique, la stabilité de l'antagoniste du VEGF dans la phase liquide étant évaluée par détermination du pourcentage de molécules natives à l'aide d'une chromatographie liquide haute performance d'exclusion stérique (SE-HPLC).

8. Formulation pharmaceutique biphasique selon la revendication 1 ou 2, dans laquelle au moins environ 85 % dudit antagoniste du VEGF dans la phase liquide a un poids moléculaire natif à 60 jours à température physiologique, d'après une détermination par chromatographie d'exclusion stérique.

9. Formulation pharmaceutique biphasique selon la revendication 1 ou 2, dans laquelle ledit antagoniste du VEGF dans la phase liquide est plus stable qu'une concentration équivalente de la phase liquide sans PEG ou dans un système biphasique fermé.

10. Formulation pharmaceutique biphasique selon la revendication 1 ou 2, ledit environnement étant un environnement physiologique.

11. Formulation pharmaceutique biphasique selon la revendication 1 ou 2, dans laquelle ladite composition pharmaceutique biphasique est contenue dans un dispositif réservoir à structure poreuse.

12. Formulation selon la revendication 1, dans laquelle ledit PEG est entièrement soluble, se trouve uniquement dans la phase liquide et est à une concentration inférieure dans ladite phase liquide que ladite protéine dans ladite phase solide.

FIGURE 1

FIGURE 2

FIGURE 3

FIGURE 4

FIGURE 5

FIGURE 6A

FIGURE 6B

Incubation Time at 37°C (Weeks)

FIGURE 7

FIGURE 8

Drug core
(insoluble phase)

Reservoir chamber
(contains drug
biphasic system)

Housing

Dissolved drug
(soluble phase)

biological fluid in

dissolved drug out

Port

FIGURE 9

FIGURE 10

FIGURE 11

FIGURE 12

FIGURE 13

FIGURE 14A

FIGURE 14B

FIGURE 15

## REFERENCES CITED IN THE DESCRIPTION

### Patent documents cited in the description

- US 2012095439 A
- WO 2008109886 A1, Desai
- WO 2012142318 A1, Desai
- US 20140170204 A1, Desai
- US 20150216829 A1, Desai
- US 20150250647 A
- US 2011046870 A

- US 20130324942 A
- US 9033911 B
- US 8623395 B
- US 20140170204, Bernards
- US 20150119807 A
- EP 2164425 A1
- US 20150119807 A1

### Non-patent literature cited in the description

- **HERRMANN et al.** *Pharmaceutical Research*, 2007, vol. 24 (8), 1527-1537
- **ATHA et al.** *Journal of Biological Chemistry*, 1981, vol. 256 (23), 12108-12117
- **ATHA et al.** Mechanism of Precipitation of Proteins by Polyethylene Glycols. *J. of Bio. Chem.*, 1981, vol. 256 (23), 12108-12117
- Drug Delivery: Engineering Principles for Drug Therapy. **W. MARK SALTZMAN**. Topics in Chemical Engineering. Oxford University Press, 2001
- **KOST et al.** *J. Biomed. Mater. Res.*, 1985, vol. 19, 1117-1133
- **ALBIN et al.** *J. Controlled Release*, 1987, vol. 6, 267-291
- **ISHIHARA et al.** *Polymer J.*, 1984, vol. 16, 625-631
- **GHADERI et al.** Production platforms for biotherapeutic glycoproteins. Occurrence, impact, and challenges of non-human sialylation. *Biotechnol Genet Eng Rev.*, 2012, vol. 28, 147-75

- **BERNARDS et al.** Nanostructured thin film polymer devices for constant-rate protein delivery. *NanoLett*, 2012, vol. 12, 5355-61
- **HE et al.** Use of a nanoporous biodegradable miniature device to regulate cytokine release for cancer treatment. *J. Control Release*, 2011, vol. 151 (3), 239-45
- **GIN ; NOBLE**. Designing the Next Generation of Chemical Separation Membranes. *Science*, 2011, vol. 332, 674-676
- **WIRTZ et al.** Transport properties of template synthesized gold and carbon nanotube membranes. *Int. J. Nanoscience*, 2002, vol. 1 (3,4), 255
- **LI et al.** Preparation and characterization of chitosan nanopores membranes for the transport of drugs. *Int. J. Pharm.*, 2011, vol. 420 (2), 371-7